# EUROPEAN PATENT APPLICATION

(11) **EP 3 842 042 A1**
(43) Date of publication of application: **30.06.2021**
(21) Application number: 19850999.4
(22) Date of filing: 19.08.2019
(51) Int. Cl.: A61K 31/375, A61K 31/19, A61K 31/191, A61K 47/52, A61K 45/06, A61P 35/00, A23L 33/10

(54) **PHARMACEUTICAL COMPOSITION FOR TREATING CANCER COMPRISING METAL ION-BOUND IONIC COMPOUND**

(30) Priority: 22.08.2018 KR 20180098145
(71) Applicant: Metafines.Co.Ltd., Seongnam-si, Gyeonggi-do 13523 (KR)
(72) Inventor: PAI, Chaul Min, Seongnam-si, Gyeonggi-do 13568 (KR)
(74) Representative: Petraz, Gilberto Luigi
(86) International application number: PCT/KR2019/010485
(87) International publication number: WO 2020/040502

(57) **Abstract**

The present invention relates to a pharmaceutical composition for treating cancer. More specifically, it relates to a pharmaceutical composition for treating cancer: which comprises an ionic compound in which two compounds selected from ascorbic acid, dichloroacetic acid and lactate are combined with one metal ion; has better therapeutic effect by overlapping and complex disturbances of cancer cell metabolism because different compounds are simultaneously uptake into cancer cells and each acts through different mechanisms on cancer cells, compared to the conventional anticancer drugs focusing on one specific mutation or cancer cell growth signal; and can more effectively inhibit the proliferation, invasion and metastasis of cancer cells because it is less susceptible to drug.

## Description

### Technical Field

The present invention relates to a pharmaceutical composition for treating cancer. More specifically, it relates to a pharmaceutical composition for treating cancer: which comprises an ionic compound in which two compounds selected from ascorbic acid, dichloroacetic acid and lactate are combined with one metal ion; has better therapeutic effect by overlapping and complex disturbances of cancer cell metabolism because different compounds are simultaneously uptake into cancer cells and each acts through different mechanisms on cancer cells, compared to the conventional anticancer drugs focusing on one specific mutation or cancer cell growth signal; and can more effectively inhibit the proliferation, invasion and metastasis of cancer cells because it is less susceptible to drug.

### Background Art

In general, there are three ways to treat cancer: surgical operation, radiation therapy and chemotherapy. Each method can be used independently for cancer treatment or a combination of two or more methods. Many early stages of cancer can be treated with surgical operation but if the cancer has advanced or spread, the surgical operation alone is difficult to treat and other methods must be used together. Radiation therapy is used to treat areas that are difficult to operate in surgery or cancers that are particularly responsive to radiation, and may be used in combination with medication before or after surgery. However, the radiation therapy has disadvantages that it induces damage to normal skin of the local area as a side effect by high energy radiation, and in the case of metastatic cancer, cancer stem cells are resistant to radiation and later recurrence or metastasis occurs. In order to treat cancer with high mortality, surgical treatment, anticancer drug therapy or radiation therapy, which is possible in early and intermediate stages of cancers, is the first priority. However, current cancer therapies generally have various side effects that, for example, it can only treat early-stage cancer or have high possibility of recurrence and destroys normal cells as well as cancer cells. In particular, in the case of patients with severe end-stage cancer, the side effects of aggressive therapy may be more severe. Therefore, treatments that slow the progression of cancer cells to reduce side effects and increase the quality of life are often selected.

In general, chemotherapy is a method of destroying or inhibiting DNA or related enzymes necessary for the proliferation of cancer cells by administering drugs orally or by injection. The chemotherapy is used as a standard therapy for treating metastatic cancer in that it can delivery drugs to cancer and treat metastatic cancer in any part of the body compared to the radiation therapy or the surgical operation. Of course, the chemotherapy cannot completely cure metastasized cancer, but it plays an important role in relieving symptoms, improving the quality of life and extending the life of the patient. However, the problem with most chemotherapeutic drugs is that it affects not only cancer cells but also normal cells, especially bone marrow, hair follicles and gastrointestinal endothelial cells, which proliferate in the human body. Therefore, cancer patients undergoing drug treatment may have side effects such as bacterial infection, spontaneous bleeding, hair loss, nausea and vomiting due to the decrease of white blood cells and platelets, which are immune cells produce in bone marrow. In addition, the drug resistance first appeared to be effective but eventually failed to treat. Immune chemotherapy, a customized therapy that uses generic technology to enhance immunity to treat cancer, has disadvantages that it is not easy to remove cancer cells only by immune function because of the increased activity of cancer cells in the advanced stage of cancer, and is not effective for patients with too much damaged immune system and patients how do not express much PD-1 (protein present on the surface of activated T cells). Further, the immune chemotherapy has the potential to present unexpected advantages, for example, since mass production is not possible, considerable costs are required and patients die during clinical trials.

Recently, the release of target anticancer drug that selectively kills only cancer cells while reducing side effects of the existing anticancer drugs and protecting normal cells is increasing. However, since it attacks only certain factors of the cancer-producing process, it is disadvantageous that, even in the case of cancers of the same kind, it is only effective for patients with specific targets. Many anticancer drugs have been developed for the treatment of cancer for many years, based on the regulation of the inhibition of the characteristics of cancer cells such as continuous cell proliferation and metastasis. However, developing anticancer drugs that effectively inhibit the proliferation of cancer cells controlled by a complex network of signaling pathways stills remains a challenge.

There is an urgent need for the development of new therapies that are easily applicable to the treatment of cancer diseases and that can effectively treat with minimal effect on normal tissues.

In order to satisfy this, a novel metabolic anticancer drug using the inherent metabolic characteristics of cancer cells has recently attracted attention. The development of genetic engineering and molecular biology techniques in the 1970s led to the discovery of cancer-causing mutations and chromosomal abnormalities, and the focus of cancer research focused on the genetic causes of cancer. Unique metabolic pathway of cancer has not been studied for a long time because it is recognized as a side effect of cancer development and not as a cause of cancer. However, as mutations in genes and various metabolites associated with cancer metabolic signals have been shown to directly induce cancer, advances in biotechnology make it possible to analyze metabolites, metabolic pathways in cancer cells resurfaced as powerful anticancer targets for treating cancer. It was first discovered that cancer cells use a metabolic pathway different from normal cells by Otto Warburg, a Nobel Prize-winning German biochemist who announced that cancer cells use a glucose metabolic pathway through a new pathway, aerobic glycolysis (Warburg effect).

Normal cells produce energy by completely oxidizing glucose to water and carbon dioxide by oxidative phosphorylation in the presence of oxygen, whereas cancer cells choose the route to oxidize glucose to pyruvate and then reduce the pyruvate to lactate (lactic acid) in an oxygen-deficient environment (Hypoxia). Thus, it was found that cancer cells consume less oxygen than normal cells and by revealing the presence of enormous amounts of lactate in the ascites of cancer patients, cancer cells use a specific metabolic pathway that produces ATP through glycolysis of consuming excess amounts of glucose than normal cells to produce lactate in excess.

In many cases, cancer cells, especially solid cancer cells, use aerobic glycolysis as a metabolic pathway for the production of energy sources (ATP), among which mechanisms include mitochondrial defects and dysfunctions, adaptation of tumors to the hypoxia microenvironment, cancer-induced signaling pathways, and abnormal expression of metabolic enzymes. The Warburg effect may also be the result of cancer cells adapting to the hypoxia microenvironment. The hypoxia environment stabilizes HIF1 (a transcription factor induced when cells lack oxygen) and induces its activation as a transcription factor by inhibiting the ubiquitinized proteosomal degradation of HIF1. On the other hand, Expression of Glucose Transporter Protein Type 1 (GLUT1) is induced by HIF1 to support glucose influx into cancer cells, and Monocarboxylate Transporter (MCT4), also a direct target of HIF1, causes lactate to be released from the inside of cancer cells to the outside by Lactate Dehydrogenase A (LDHA), which converts pyruvate into lactate. In addition, HIF1 induces the expression of Pyruvate Dehydrogenase Kinase, which inhibits Pyruvate Dehydrogenase (PDH), an enzyme that converts pyruvate to Acetyl-Co, thereby closing the pathway to oxidative phosphorylation. Therefore, HIF1 is a very important factor that induces Warburg effect through direct expression control of various factors related to glucose influx and glycolysis.

Meanwhile, cancer cells quickly release lactate, the end product of aerobic glycolysis, to prevent acidification on its own. The released lactate inactivates cytokine, which is produced in cytotoxic T and dendritic cells and plays an important role in anti-cancer effects, inhibits the expression of NKp46 (a recognition receptor of natural killer cell (NK cell)), an activator of natural killer cells, and promotes production of immune-suppressing substances, thereby inhibiting apoptosis of cancer cells. In addition, endothelial cells around cancer cells induce the expression of IL-8 (protein acting as a chemoattractant that activates inflammatory cells and attracts them to the inflamed area) and VEGF (vascular endothelial growth factor) by introducing the released lactate, and promote endothelial cell migration, thereby inducing angiogenesis. This metabolic reprogramming of cancer cells is an evolutionarily chosen metabolic transformation strategy to produce precursors such as nucleotides, lipids and amino acids, which are necessary for the synthesis of cellular components of rapidly growing cancer cells rather than simply producing ATP, and it is understood that continuously growing cancer cells use this metabolic pathway strategically. As such, the development of cancer by the existing various carcinogenic factors that induce the formation and growth of cancers is closely related to cancer cell metabolism, and reprogramming of cell metabolism may be an important anticancer target for effective treatment of cancer. In order to selectively remove tumors by understanding this specific metabolic signal of cancer cells, the development of metabolism targeted drugs for controlling the glucose metabolism of cancer cells has been intensively performed. In particular, the development of cancer drugs using various drugs that have been previously used for glucose metabolism and infectious diseases is being conducted.

### [Prior Art Documents]

### [Patent documents]

(Patent Document 1) Korean Laid-open Patent Publication No. 10-2016-0082918 (2016. 07. 11.)
(Patent Document 2) U.S. Laid-open Patent Publication No. US2011/0117210 (2011. 05. 19.)
(Patent Document 3) Korean Laid-open Patent Publication No. 10-2005-0058278 (2005. 06. 16.)

### Disclosure

### Technical Problem

As a result of various studies to understand cancer cell-specific metabolic signals and to develop cancer-targeted therapeutics and treatment methods that can selectively remove tumors, the inventors of the present invention completed the present invention by finding out that proliferation, invasion, metastasis and the like of cancer cells can be effectively suppressed in the case of using an ionic compound in which two compounds selected from ascorbic acid, dichloroacetic acid and lactate are combined with one metal ion selected from Ca, Zn, Mg and Fe.

Therefore, it is an object of the present invention to provide a pharmaceutical composition for treating cancer, which comprises an ionic compound in which two compounds selected from ascorbic acid, dichloroacetic acid and lactate are combined with one metal ion selected from Ca, Zn, Mg and Fe as an active ingredient.

### Technical Solution

In order to achieve the above objects, the present inventors have focused on the major metabolic pathways unique to cancer cells in order to develop methods for effectively inhibiting the proliferation and metastasis of cancer cells.

The first notable metabolic pathway of cancer cells is aerobic glycolysis. Cancer cells mainly use aerobic glycolysis, which does not require oxygen, rather than oxidative phosphorylation, an energy metabolic process that requires oxygen. Therefore, cancer cells can survive in Hypoxia environment, such as solid cancers, in which normal cells cannot survive, and apoptosis control processes originating from the mitochondria are inactivated.

The second notable metabolic pathway of cancer cells involves large amounts of lactate produced through the aerobic glycolysis. The lactate is rapidly released out of the cancer cells to prevent acidification of the cancer cells themselves and makes the surrounding environment of the cancer cells acidic (Acidosis). The acidified environment inhibits the activity of NK and CTL cells, and eventually, angiogenesis, cancer cell metastasis and immunosuppression are induced.

Third, it was noted that calcium plays an important role in cytosolic calcium buffering as an essential factor for the survival and proliferation of cancer cells, and also has a significant effect on apoptosis and autophagy in cells involved in the production of active oxygen species. In particular, calcium is known to be maintained at low concentrations in cancer cells. Reducing the supply of calcium to the mitochondria in cancer cells inhibits cancer cell proliferation due to energy depletion, while increasing the supply of calcium overloads the mitochondria and kills the cancer cells. Therefore, it was noted that cancer cells respond more sensitively to calcium than normal cells, and when the homeostasis of calcium in the cancer cells is destroyed, the cancer cells die beyond the inhibition of cancer cell proliferation.

The present invention provides a pharmaceutical composition for treating cancer comprising an ionic compound, effectively acting on major metabolic pathways specific to such cancer cells, in which two compounds selected from ascorbic acid, dichloroacetic acid and lactate are combined with one metal ion selected from Ca, Zn, Mg and Fe.

### Advantageous Effects

The pharmaceutical composition for treating cancer according to the present invention can be used as a metabolic anticancer drug comprising an ionic compound in which two compounds selected from ascorbic acid, dichloroacetic acid and lactate are combined with one metal ion selected from Ca, Zn, Mg and Fe as an active ingredient, and it can effectively suppress the proliferation of cancer cells.

Further, since the pharmaceutical composition for treating cancer according to the present invention includes compounds having different mechanisms such as cancer cell growth inhibitory compounds and cancer cell metastasis inhibitory compounds, it can simultaneously act on major metabolic enzymes, causing overlapping and complex disturbances of cancer cell metabolism, resulting in exerting its effects simultaneously at the cellular level unlike the conventional anticancer drugs, which focus on one specific mutation or blocking metabolic processes.

Further, the pharmaceutical composition for treating cancer according to the present invention is an ionic compound that can enhance the uptake of cancer cells when administered into the body.

Further, the pharmaceutical composition for treating cancer according to the present invention improves cancer cell uptake by converting acidic compounds into neutral metal salts, is less susceptible to drug resistance, and can effectively inhibit the action of cancer cells such as proliferation, invasion and metastasis.

Further, the present invention can provide a method for treating cancer and a method for suppressing cancer metastasis using the pharmaceutical composition for treating cancer according to the present invention.

Further, the present invention can provide a food composition for suppressing cancer metastasis or for improving cancer comprising the pharmaceutical composition for treating cancer according to the present invention.

Further, the pharmaceutical composition for treating cancer according to the present invention has low side effects in the body, so it can be used as an additive in food and can be administered in high doses.

### Description of Drawings

FIG. 1a is a graph showing the calcium concentration in cancer cells treated with the calcium salts of Examples 1 to 3.
FIG. 1b is an image showing calcium in cancer cells treated with the calcium salts of Examples 1 to 3.
FIG. 2 is a graph showing the lactate concentration in cancer cells treated with the calcium salts of Examples 1 to 3.
FIG. 3 is a graph showing the lactate concentration released from cancer cells treated with the calcium salts of Examples 1 to 3.
FIG. 4 is a graph showing the ascorbic acid concentration treated with the calcium salts of Examples 1 and 2 and Comparative Examples 1 and 2.
FIG. 5 is a graph showing pH in cancer cells treated with the calcium salts of Examples 1 to 3.
FIG. 6 is a graph showing pyruvate concentration in cancer cells treated with the calcium salts of Examples 1 to 3.
FIG. 7 is a graph showing the α-KG concentration in cancer cells treated with the calcium salts of Examples 1 to 3.
FIG. 8 is a graph showing the expression levels of PARP, β-catenin, VEGF and β-actin expressed from cancer cells treated with the calcium salts of Examples 1 to 3.
FIG. 9 is a graph showing the expression level of active oxygen expressed from cancer cells treated with the calcium salts of Examples 1 to 3.
FIG. 10 is a graph showing apoptosis of cancer cells treated with the calcium salts of Examples 1 to 3.
FIG. 11 is an image confirming colony-forming ability by treating the calcium salts of Examples 1 to 3 to colorectal cancer cell lines.
FIG. 12 is a graph confirming colony-forming ability by administrating the calcium salts of Examples 1 to 3 in combination with the conventional 5-FU anticancer drug to colorectal cancer cell lines.
FIG. 13 is a graph confirming the co-delivery effect by treating the calcium salts of Example 1 and 5-FU anticancer drug together to HCT-116, a colorectal cancer cell line.
FIG. 14 is a graph confirming the co-delivery effect by treating the calcium salts of Example 2 and 5-FU anticancer drug together to HCT-116, a colorectal cancer cell line.
FIG. 15 is a graph confirming the co-delivery effect by treating the calcium salts of Example 1 and SN-38 anticancer drug together to HCT-116, a colorectal cancer cell line.
FIG. 16 is a graph confirming the co-delivery effect by treating the calcium salts of Example 2 and SN-38 anticancer drug together to HCT-116, a colorectal cancer cell line.
FIG. 17 is a graph confirming the co-delivery effect by treating the calcium salts of Example 1 and Paclitaxel anticancer drug together to HCT-116, a colorectal cancer cell line.
FIG. 18 is a graph confirming the co-delivery effect by treating the calcium salts of Example 2 and Paclitaxel anticancer drug together to HCT-116, a colorectal cancer cell line.
FIG. 19a is imaging results obtained after calcium salts of Example 1 was injected into the mouse model (DLD-1 orthotopic model) and dissected 1 week later, and FIG. 19b is a graph showing the weight of cancer tissue dissected 1 week after calcium salts of Example 1 were administered to the mouse model (DLD-1 orthotopic model).
FIG. 20 is an image of growth saturation degree obtained by date with luminescence imaging after calcium salts of Examples 1 to 3 were administered to the mouse model implanted with A549/LUC cells into the lung.
FIG. 21 is a graph showing the image of FIG. 20 by measuring Region Of Interest (ROI) which is a program of IVIS spectrum (Xenogen).
FIG. 22 is a graph showing the survival rate measured after administration of the calcium salts of Examples 1 to 3 to the mouse model implanted with A549/LUC cells into the lung.

### Mode for carrying out the invention

The present invention provides a pharmaceutical composition for treating cancer, which comprises an ionic compound in which two compounds selected from ascorbic acid, dichloroacetic acid and lactate are combined with one metal ion selected from Ca, Zn, Mg and Fe as an active ingredient.

The ascorbic acid (L-ascorbic acid) is vitamin C, which has already been identified as a non-toxic anticancer drug through the study of Nobel prize laureate Linus Pauling. Further, ascorbic acid does not show any particular toxicity even when the human body is administered in excess (50 g or more), and its glucose-like structure can competitively inhibit glucose addition of cancer cells. Meanwhile, mega dose of ascorbic acid can induce cancer cell necrosis by dropping glutathione or NADPH in cancer cells and generating reactive oxygens (ROS). Further, ascorbic acid induces cancer cells to differentiate into normal cells and inhibits the spread of cancer cells to peri-cancerous tissues through collagen synthesis and blocking of enzymes that help cancer metastasis. In addition, it can destroy cell membranes and reduce cancer pain by entering into cancer cells, increase immunity by detoxing important organs of cancer patients, and inhibit neovascularization of cancer. Further, it can play an important role in cancer prevention and treatment by increasing the efficacy of other anticancer drug treatments and radiation therapy and reducing side effects.

Meanwhile, at low concentrations of ascorbic acid, no apoptosis of cancer cells is observed, but at the G1 stage of cancer cells, complete growth is inhibited, p53 level is increased, CDK2 activity is inhibited, and p38MARK activation and COX-2 expression can be decreased.

At high concentrations of ascorbic acid, it is possible to induce apoptosis in cancer cells by decreasing the potential of the mitochondrial membrane, decreasing the expression of Tf transporters, decreasing iron uptake, and increasing reactive oxygens (ROS) in cancer cells.

When the ascorbic acid is combined with an appropriate metal ion, its stability in the body is increased and its uptake into cancer cells is increased. Therefore, it is more effective than the anticancer effect of the existing ascorbic acid, and can induce apoptosis of cancer cells even at relatively low concentrations.

In general, in hypoxia state, in cancer cells, hypoxia-inducible factor-1 (HIF-1) is activated to express pyruvate dehydogenase kinase and pyruvate dehydrogenase complex is inhibited by the expressed pyruvate kinase. As a result, pyruvate is not converted into acetyl-CoA, which leads to the accumulation of pyruvate and a decrease in the energy synthesis of mitochondria. Thus, when pyruvate accumulates in excess, pyruvate is converted to lactate, causing the lactate to be accumulated around cancer cells. In summary, when cancer cells become hypoxic, accumulation of lactate begins with the expression of pyruvate kinase.

The dichloroacetic acid is not toxic and may block the aerobic glycolysis pathway described above. Further, the accumulation of lactate can be suppressed by inhibiting the expression of pyruvate kinase. In addition, reconstitution of Tricarboxylic Acid Cycle (TCA cycle) can induce glucose metabolism reprogramming (i.e., normalization of mitochondrial metabolism) by mitochondrial respiration acceleration.

On the other hand, by combining with an appropriate metal ion, the dichloroacetic acid can kill cancer cells by improving the anticancer effect of the existing dichloroacetic acid, and inducing normalization of mitochondrial metabolism and reactive oxygen (ROS).

In addition, by combining with an appropriate metal ion, the dichloroacetic acid can inhibit the tumor acidosis by reducing the accumulation of lactate.

The lactate includes lactic acid, D-lactate and L-lactate, and it means to include D-lactic acid and L-lactic acid.

By combining the lactate with an appropriate metal ion, the lactate in cancer cells may be excessively accumulated to activate L-lactate dehydrogenase B (LDHB; an enzyme that coverts lactate or lactic acid to pyruvate, and at the same time, an enzyme that converts NAD+ to NADH) or to inhibit L-lactate dehydrogenase A (LDHA; LDHB reverse reaction enzyme), thereby inhibiting Monocarboxylate transporters (MCT) expression.

Herein, "inhibition of LDHA" or "activation of LDHB" refers to the conversion of lactate to pyruvate. Further, "inhibition of MCT expression" means inhibiting the expression of MCT involved in the inflow and discharge of lactate, thereby activating the expression of NKp46 and activating apoptosis of cancer cells.

Further, by combining the lactate with an appropriate metal ion, it can be administered into cancer cells to acidify the inside to induce apoptosis.

The metal ion may be one selected from Ca, Zn, Mg and Fe. Preferably, the metal ion is Ca, Mg or Fe, and more preferably Ca²⁺ ion, but not limited thereto.

In this case, the Ca²⁺ ion (calcium ion) affects the calcium homeostasis of cancer cells. It can generate excess reactive oxygen in cancer cells by inducing calcium accumulation in the mitochondria, and can cause cancer cell apoptosis by the generated reactive oxygen.

To be more specific, in the mitochondria, which are responsible for the energy production of cancer cells, calcium binds directly to alpha-ketoglutarate dehydrogenase and is an important factor for the normal operation of the TCA cycle. It is known that loss of calcium homeostasis is particularly important to reduce cancer cells. When the calcium concentration is excessively increased in cancer cells, endonuclease and many proteases are activated. It leads to mitochondrial metabolism disturbance, release of cytochrome C, activation of caspase 9 and subsequent activation of caspase 3 and caspase 7. This leads to apoptosis.

Herein, "ionic compound" refers to a compound in which ions with opposite charges due to electrostatic forces are formed through ionic bonds and this compound generally exhibits electrical neutrality.

The ionic compound included in the pharmaceutical composition according to the present invention may preferably be any one of calcium salts of ascorbic acid and dichloroacetic acid, calcium salts of ascorbic acid and lactate, calcium salts of dichloroacetic acid and lactate, magnesium salts of ascorbic acid and dichloroacetic acid, magnesium salts of ascorbic acid and lactate, magnesium salts of dichloroacetic acid and lactate, iron salts of ascorbic acid and dichloroacetic acid, iron salts of ascorbic acid and lactate, and iron salts of dichloroacetic acid and lactate. More preferably, it may be any one of calcium salts of ascorbic acid and dichloroacetic acid, calcium salts of ascorbic acid and lactate, and calcium salts of dichloroacetic acid and lactate, but not limited thereto.

Herein, the "calcium salts" refers to ionic compounds produced or synthesized in the form of compounds combined with calcium ions, the "magnesium salts" refers to ionic compounds produced or synthesized in the form of compounds combined with magnesium ions, and the "iron salts" refers to ionic compounds produced or synthesized in the form of compounds combined with iron ions.

The pharmaceutical composition according to the present invention may be used for treatment in combination with radiation or with anticancer drug. In general, it decreases the expression of PARP, HIF-1α and VEGF that give a cancer cell resistance to radiation in case of radiation. Thus, in case of administration of the composition in combination with radiation, it improves the anticancer activity of radiation. Therefore, it is possible to obtain an equivalent anticancer effect with a decreased amount of radiation as compared with the conventional case. In this case, a dose of radiation that can be used is not particularly limited, but may be 2 to 10 Gy per day. The radiation may be irradiated once a day or may be irradiated over several days by dividing the dose.

The pharmaceutical composition according to the present invention, which comprises an ionic compound in which two compounds selected from ascorbic acid, dichloroacetic acid and lactate are combined with one metal ion selected from Ca, Zn, Mg and Fe, can be simultaneously uptake in cancer cells to exert its efficacy simultaneously without offsetting the respective anticancer effects of the two compounds. These effects may be superior to Combitherapy of the conventional anticancer drugs.

On the other hand, when the pharmaceutical composition according to the present invention and anticancer drug are co-administered, the anticancer effect may be superior to the administration of the anticancer drug alone.

In this case, the anticancer drug that can be administered in combination with the pharmaceutical composition according to the present invention is not particularly limited as long as it is not directly involved in the overall metabolism of a cancer cell. For example, the anticancer drug may be known anticancer drugs such as Imatinib, 5-Florouracil (5-FU), Irinotecan, Sunitinib, Oxaliplatin, Paclitaxel, Lapatinib, Trastuzumab (Herceptin), Gefitinib, Erlotinib, Methotrexate, Carboplatin, Docetaxel, Everolimus, Sorafenib, carbonic anhydrase inhibitor, monocarboxylate transporter inhibitor, Pembrolizumab, Atezolizumab, PD-1family anticancer drug, Nivolumab, Poly (ADP-ribose) polymerase 1 (PARP-1) inhibitor, Poly (ADP-ribose) polymerase 2 (PARP-2) inhibitor, Olaparib, Rucaparib, Niraparib, Bevacizumab and VEGF inhibitor as well as other anticancer drugs known as having an anticancer activity.

In the present invention, the cancer may be a cancer whose proliferation, infiltration, metastasis and the like can be suppressed by disturbing metabolism thereof. For example, it may be lung cancer, breast cancer, colorectal cancer, stomach cancer, brain cancer, pancreatic cancer, thyroid cancer, skin cancer, bone marrow cancer, lymphoma, uterine cancer, cervical cancer, kidney cancer and melanoma.

The pharmaceutical composition of the present invention may be prepared in the form of a pharmaceutical composition for treating cancer, which further includes appropriate carriers, excipients or diluents generally used in preparation of a pharmaceutical composition. Specifically, the pharmaceutical composition may be formulated, according to a traditional method, into a form of oral dosage forms such as powder, granule, tablet, capsule, suspension, emulsion, syrup, aerosol and oral patch, external preparation, patch for external use, suppository and sterile injectable solutions.

In the present invention, the carriers, excipients and diluents which may be included in the pharmaceutical composition may be lactose, dextrose, sucrose, sorbitol, mannitol, xylitol, erythritol, maltitol, starch, acasia rubber, alginate, gelatin, calcium phosphate, calcium silicate, cellulose, methyl cellulose, amorphous cellulose, polyvinyl pyrrolidone, water, methylhydroxybenzoate, propylhydroxybenzoate, talc, magnesium stearate, mineral oil and the like. When formulated, it may be prepared using diluents or excipients such as fillers, bulking agents, binders, wetting agents, disintegrating agents and surfactants commonly used. The solid formulations for oral administration may include tablets, depots, pills, powders, granules, capsules, oral patches and the like. The solid formulations may be prepared by mixing at least one excipients, for example, starch, calcium carbonate, sucrose or lactose, gelatin and the like with the extracts and fractions thereof. Further, in addition to such general excipients, lubricants such as magnesium stearate or talc may also be used. The liquid formulations for oral administration may include suspensions, solutions for internal use, emulsions, syrups and the like. In addition to general diluents such as water and liquid paraffin, different excipients such as wetting agents, flavors, fragrances, preserves and the like may be included. The formulations for parenteral administration may include sterile aqueous solutions, non-aqueous solvents, suspensions, emulsions, lyophilized preparations, patches for external use, or suppositories. The non-aqueous solvents and the suspensions may include propylene glycol, polyethylene glycol, vegetable oil such as olive oil, injectable ester such as ethyloleate, etc and the like. The base for suppositories may include witepsol, macrogol, tween 61, cacao butter, laurin butter, glycerogelatin and the like.

The amount of the ionic compound included in the pharmaceutical composition of the present invention is not particularly limited, but it may be between 0.0001 wt% and 50 wt%, and more preferably between 0.01 wt% and 20 wt%, based on the total weight of the final composition. The concentration of the metal ion included in a single dose of the pharmaceutical composition may be 0.1 mM to 300 mM.

The pharmaceutical composition of the present invention may be administered in a pharmaceutically effective amount, and as used herein, the term "pharmaceutically effective amount" refers to an amount sufficient to treat or prevent diseases, at a reasonable benefit/risk ratio applicable to any medical treatment or prevention. The effective dosage level may be determined depending on severity of the disease, activity of the drug, a patient's age, body weight, health and sex, sensitivity to the drug, administration time, administration route, and excretion rate of the composition of the present invention, duration of treatment, drugs used simultaneously or in combination with the composition of the present invention, and other factors known in the medical field. The pharmaceutical composition of the present invention may be administered alone or in combination with other known anticancer drugs or components known as having an anticancer activity. It is important to administer the composition in the minimum amount that can exhibit the maximum effect without causing side effects, in consideration of all the above factors.

The dosage of the pharmaceutical composition of the present invention may be determined by those skilled in the art in consideration of the purpose of use, severity of the disease, a patient's age, body weight, sex, anamnesis, the kind of material used as an active ingredient and the like. For example, the pharmaceutical composition of the present invention may be administered at a dosage of about 1 ng to about 2,000 mg/kg per adult, or preferably 1 mg to about 400 mg/kg per adult. The administration frequency of the composition of the present invention is not particularly limited, but it may be once or a few divided doses a day. The dosage or the administration frequency does not limit the scope of the present invention in any way.

In another aspect, the present invention provides a method of treating cancer comprising the step of administering a pharmaceutically effective amount of the pharmaceutical composition to a subject having cancer.

As used herein, the term "subject" includes all mammals including mice, livestock and humans, and farm fish that have cancer, without limitations.

The term "treatment" used herein refers to all activities to alleviate or improve the symptoms of cancer by administering the pharmaceutical composition including the ionic compound of the present invention as an active ingredient to a subject having cancer.

In the method of treating cancer of the present invention, the kinds of cancer to be treated are the same as described above.

The composition can be administered in a single or multiple dosage form. In this case, the composition may be formulated into liquid, powder, aerosol, injection, fluid transfusion (intravenous drip), capsule, pill, tablet, suppository or patch.

The pharmaceutical composition for treating cancer of the present invention may be administered via any of common routes as long as it is able to reach a target tissue.

The pharmaceutical composition of the present disclosure may be administered intraperitoneally, intravenously, intramuscularly, subcutaneously, intradermally, in the form of a transdermal patch, orally, intranasally, intrapulmonarily or intrarectally depending on the purpose, but not particularly limited thereto. However, the pharmaceutical composition may be administered in a non-formulated form for oral administration, and since the metal lactate salts may be denatured by gastric acid upon oral administration, an active ingredient of a composition for oral administration should be coated or formulated for protection against degradation in the stomach, or orally administered in the form of a patch for oral administration. Further, the composition may be administered in the form of long acting injection to maximize efficacy in the injection administration. In addition, the composition may be administered using a certain apparatus capable of transporting the active ingredient into a target cell.

Further, the pharmaceutical composition of the present invention may be formulated as a sustained release formulation to effectively maintain the concentration of the drug, i.e., an ionic compound, in the body. For example, the rate at which the drug is released in the body can be controlled by administration once a day or once a week while maintain the efficacy. In this case, the sustained release formulation may include carriers, excipients or diluents as described above

In another aspect, the present invention provides a pharmaceutical composition for suppressing cancer metastasis, which comprises an ionic compound in which two compounds selected from ascorbic acid, dichloroacetic acid and lactate are combined with one metal ion selected from Ca, Zn, Mg and Fe.

The ionic compound provided in the present invention can inhibit various properties that can induce cancer cell metastasis such as cancer cell metastasis, invention, neovascularization, colony forming ability. Therefore, it can be used as an active ingredient of a pharmaceutical composition for suppressing cancer metastasis.

Further, the ionic compound and the metal ion are the same as described above.

In this case, a metastasis-suppressed target cancer is the same as defined above. For example, the pharmaceutical composition for suppressing cancer metastasis may be used for suppressing the occurrence of at least one metastatic cancers selected from the group consisting of metastatic lung cancer, breast cancer, colorectal cancer, stomach cancer, brain cancer, pancreatic cancer, thyroid cancer, skin cancer, bone marrow cancer, lymphoma and melanoma.

In another aspect, the present invention provides a method of suppressing cancer metastasis comprising the step of administering a pharmaceutically effective amount of the pharmaceutical composition to a subject expected cancer metastasis.

In the present invention, the term "metastasis" refers to a condition in which cancer or a malignant tumor has spread to other tissues away from the organ that develop the cancer or malignant tumor.

When the ionic compound provided in the present invention is administered, the metastasis can be suppressed.

In the method of suppressing cancer metastasis of the present invention, the kinds of cancer to be targeted for suppressing metastasis, the form of a drug to be administered, and the route of drug administration are the same as described above.

In another aspect, the present invention provides a for preventing or improving fatigue related to cancer comprising the ionic compound as an active ingredient.

Herein, the fatigue related to cancer is one of the most frequent side effects during or after cancer treatment, and for example, it may include Cancer-related fatigue (CRF). Herein, the cancer-related fatigue refers to symptoms that are painful, persistent, independent of recent activity, and interfere with daily functioning with a subjective sense of tiredness and exhaustion from cancer and its treatment.

Generally, it is known that cancer patients' lack of ascorbic acid increases brain-blood barrier permeability, causing neurotoxic substances or viruses to easily invade the brain, causing various fatigue syndromes. Further, lack of ascorbic acid increases neurotoxic substances such as adrenochrome and noadrenochrome, causing organ damage.

Accordingly, the ionic compound according to the present invention is made by combining two or more compounds containing ascorbic acid with a metal ion. When the compound is applied to cancer patients, it has the effect of restoring immune function, reducing muscle pain, and reducing fatigue caused by stress, thereby preventing or improving cancer fatigue syndrome. This effect can also increase the survival rate of cancer patients.

In another aspect, the present invention provides a food composition for improving cancer comprising the ionic compound as an active ingredient.

In this case, the ionic compound is the same as described above.

The ionic compound can be taken in the form of a food that can be daily eaten and can promote the improvement of cancer. In this case, the amount of calcium salts included in the food may be between 0.001 wt% and 10 wt%, or between 0.1 wt% and 1 wt%, based on the total weight of the food composition, but not particularly limited thereto. If the food is a beverage, the calcium salts may be included at a ratio of 1 g to 10 g or 2 g to 20 g per 100 ml.

Further, the composition may further include additional components which have been typically used in a food composition to improve smell, taste, appearance and the like. For example, the components may be vitamins A, D, E, B1, B2, B6, B12, niacin, biotin, folate, panthotenic acid and the like. Further, the composition may further include minerals such as Zn, Fe, Ca, Cr, Mg, Mn, Cu and the like. Moreover, the composition may further include amino acids such as lysine, tryptophan, cysteine, valine and the like. In addition, the composition may further include food additives such as preservatives (potassium sorbate, sodium benzoate, salicylic acid, dehydro sodium acetate, etc.), disinfectants (bleaching powder, higher bleaching powder, sodium hypochlorite, etc.), antioxidants (butylhydroxyanisole (BHA), butylhydroxytoluene (BHT), etc.), coloring agents (tar color, etc.), color-developing agents (sodium nitrite, sodium acetate, etc.), bleaching agents (sodium sulfite), seasonings (monosodium glutamate (MSG), etc.), sweeteners (dulcin, cyclemate, saccharin, sodium, etc.), flavors (vaniline, lactones, etc.), swelling agents (alum, potassium D-hydrogen tartrate, etc.), fortifiers, emulsifiers, thickeners (adhesive pastes), film-forming agents, gum base agents, antifoaming agents, solvents, improvers, etc. The food additives may be selected according to the kind of food and used in an appropriate amount.

Meanwhile, functional foods for improving cancer may be manufactured using the food composition for improving cancer including the ionic compound.

Specifically, processed foods capable of improving cancer may be manufactured using the food composition. Examples of the processed foods may be manufactured as functional foods in the form of cookies, beverages, alcoholic beverages, fermented foods, canned foods, milk-processed foods, meat-processed foods, or noodles. In this case, examples of the cookies include biscuits, pies, cakes, breads, candies, jellies, gums, cereals (meal substitutes such as grain flakes). Examples of beverages include drinking water, carbonated soft drinks, functional isotonic drinks, juices (e.g., apple-, pear-, grape-, aloe-, tangerine-, peach-, carrot-, tomato juices, etc.), sweet rice drinks and the like. Examples of alcoholic beverages include refined rice wine, whisky, soju (Korean distilled spirits), beer, liquors, fruits wine and the like. Examples of fermented foods include soy sauce, bean paste, red pepper paste and the like. Examples of canned foods include seafood canned foods (e.g., canned tuna, mackerel, mackerel pike, conch, etc.), livestock canned foods (canned beef, pork, chicken, turkey, etc.), and agricultural canned foods (canned corn, peach, pineapple, etc.). Examples of milk-processed foods include cheese, butter, yogurt and the like. Examples of meat-processed foods include pork cutlets, beef cutlets, chicken cutlets, sausages, sweet and sour pork, nuggets, neobiani and the like. Examples of noodles include dried noodles, plain noodles, ramen, udon noodles, Korean cold noodles, sealed and packed fresh noodles and the like. Additionally, the composition may be used for manufacturing retort foods, soups and the like.

As used herein, the term "functional food", which has the same meaning as the term "food for special health use (FoSHU)", refers to a food with high effects in medicinal and medical treatment, processed so as to efficiently exhibit a body modulating function as well as provide nutrients. The functional food may be manufactured in various forms including tablets, capsules, powders, granules, liquids, pills and the like, in order to obtain useful effects for the improvement of cancer.

Hereinafter, the present disclosure will be described in more detail with reference to the following examples. However, these examples are provided for illustrative purposes only but not intended to limit the scope of the present disclosure.

### Preparation Example 1-1: Preparation of calcium salts of dichloroacetic acid and ascorbic acid

129 mg dichloroacetic acid was dissolved in 125 ml distilled water to prepare dichloroacetic acid solution, and 176 mg ascorbic acid was dissolved in 125 ml distilled water to prepare ascorbic acid solution. The ascorbic acid solution was slowly added to the dichloroacetic acid solution while stirring. Then, 105 mg calcium carbonate (CaCO₃) was slowly added thereto while stirring at room temperature for 30 min, and then reacted until no more CO₂ was generated while raising the reaction temperature slowly up to 60°C. The resulting product was dried by a rotary evaporator and a vacuum oven and unreacted substances were removed by diethyl ether, followed by filtration, drying and pulverization to obtain powder type calcium salts of dichloroacetic acid and ascorbic acid. All reaction was conducted in the presence of nitrogen.

### Preparation Example 1-2: Preparation of calcium salts of ascorbic acid and lactate

90 mg L-lactic acid was dissolved in 125 ml distilled water to prepare lactic acid solution, and 176 mg ascorbic acid was dissolved in 125 ml distilled water to prepare ascorbic acid solution. The ascorbic acid solution was slowly added to the lactic acid solution while stirring. Then, 105 mg calcium carbonate (CaCO₃) was slowly added thereto while stirring at room temperature for 30 min, and then reacted until no more CO₂ was generated while raising the reaction temperature slowly up to 60°C. The resulting product was dried by a rotary evaporator and a vacuum oven and unreacted substances were removed by diethyl ether, followed by filtration, drying and pulverization to obtain powder type calcium salts of ascorbic acid and lactate. All reaction was conducted in the presence of nitrogen.

### Preparation Example 1-3: Preparation of calcium salts of dichloroacetic acid and lactate

640 mg dichloroacetic acid and 450 mg L-lactic acid were dissolved in 10 ml distilled water while stirring, and then 500 mg calcium carbonate (CaCO₃) was slowly added thereto while stirring at room temperature for 30 min. The resulting product was dried by a rotary evaporator and a vacuum oven and unreacted substances were removed by diethyl ether, followed by filtration, drying and pulverization to obtain powder type calcium salts of dichloroacetic acid and lactate.

### Example 1

Calcium salts prepared according to Preparation Example 1-1 in which dichloroacetic acid and ascorbic acid are combined with calcium ions.

### Example 2

Calcium salts prepared according to Preparation Example 1-2 in which ascorbic acid and lactate are combined with calcium ions.

### Example 3

Calcium salts prepared according to Preparation Example 1-3 in which dichloroacetic acid and lactate are combined with calcium ions.

### Test Example 1: Effect of calcium salts on uptake and pH change in cancer cells

After calcium salts of Examples 1 to 3 were treated to cancer cells, respectively, changes in intracellular calcium concentration, lactate concentration, ascorbic acid concentration and pH were analyzed to predict inflow level of each calcium salts.

### Test Example 1-1: Change in calcium level

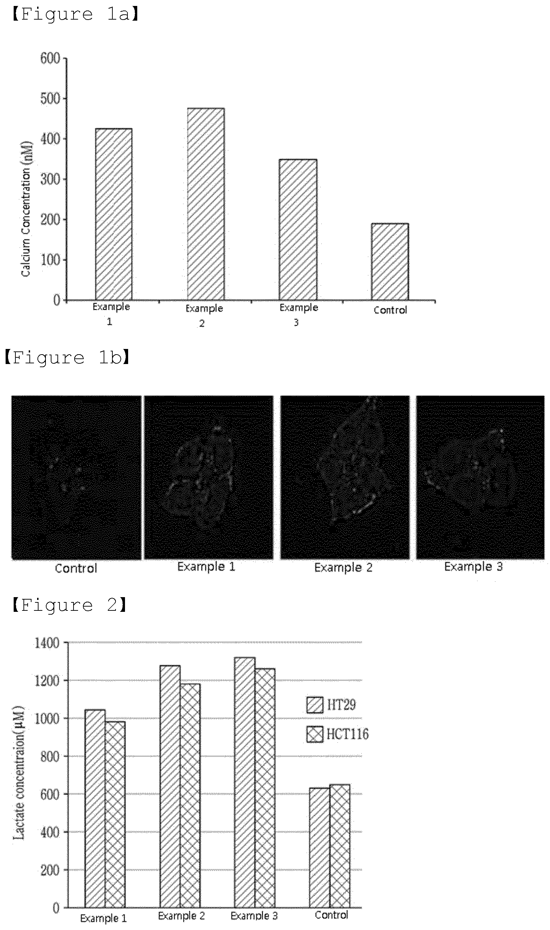

1 mM of the calcium salts of Examples 1 to 3 were treated to human colorectal cancer cell line (HCT-116) of 5 x 10⁶ cells, which was cultured in cancer cell culture medium (RPMI1640 medium containing 10% FBS and 1% penicillin/streptomycin) at a condition of 37°C and 5% CO₂, respectively, and then cultured for 24 hours. The cultured cancer cells were pulverized with a homogenizer and centrifuged, and the concentration of calcium contained in the lysate was measured using a calcium assay kit (Biovision, SanFrancisco, CA). The results are shown in FIG. 1a. In this case, cancer cells not treated with the calcium salts were used as a control.

Further, in order to observe change in calcium level by fluorescence imaging, human colorectal cancer cell line (HCT-116) of 3 x 10⁴ cells were spread on a 6-well plate and cultured for 24 hours. The calcium salts of Examples 1 to 3 were treated there to at a concentration of 1 mM, respectively, cultured for 4 hours, washed twice with DPBS, and cultured with Fluo 4-AM for 40 min. In order to evaluate the intracellular calcium concentration, fluorescence for intracellular calcium concentration was measured using FACSCanto™ II flow cytometer (Becton-Dickinson, Franklin Lakes, Nj, USA), primary argon laser, and the results are shown in FIG. 1b. In this case, cancer cells not treated with the calcium salts were used as a control.

As shown in FIG. 1a and FIG. 1b, the calcium concentration was increased in the cancer cells treated with the calcium salts of Examples 1 to 3. Accordingly, it was confirmed that the composition for treating cancer according to the present invention, which comprises an ionic compound combined with a metal ion can penetrate into cancer cells.

### Test Example 1-2: Change in lactate level

1 mM of the calcium salts of Examples 1 to 3 were treated to human colorectal cancer cell lines (HCT-116 and HT-29) of 5 x 10⁶ cells, which were cultured in cancer cell culture medium (RPMI1640 medium containing 10% FBS and 1% penicillin/streptomycin) at a condition of 37°C and 5% CO₂, respectively, and then cultured for 24 hours. The cultured cancer cells were pulverized with a homogenizer and centrifuged, and the concentration of lactate contained in the lysate was measured using a lactate assay kit (Biovision, SanFrancisco, CA). The results are shown in FIG. 2. In this case, cancer cells without any treatment were used as a control.

As shown in FIG. 2, the lactate concentration was increased in the cancer cells treated with the calcium salts of Examples 1 to 3. Accordingly, it was confirmed that the composition for treating cancer according to the present invention, which comprises an ionic compound combined with a metal ion, can penetrate into cancer cells and increase the lactate concentration.

### Test Example 1-3: Change in level of extracellular lactate released by cancer cells

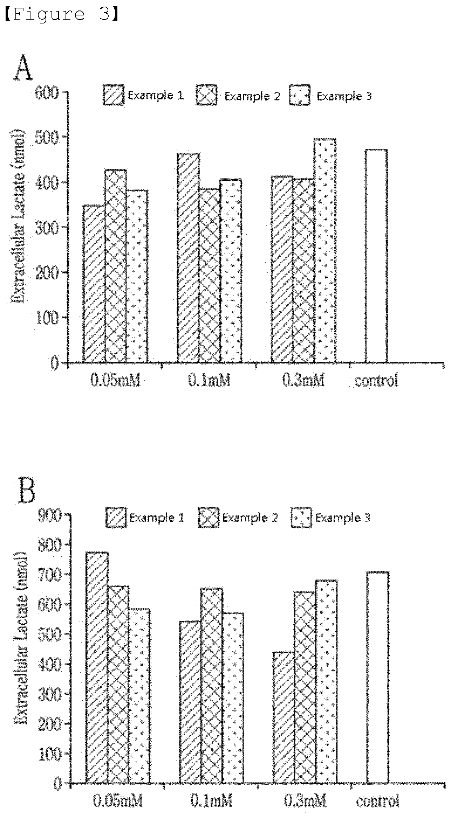

Human colorectal cancer cell lines (HCT-116 and HT-29) of 5 x 10⁵ cells, which were cultured in cancer cell culture medium (RPMI1640 medium containing 10% FBS and 1% penicillin/streptomycin) at a condition of 37°C and 5% CO₂, were spread on a 6-well plate and then cultured for 24 hours. The calcium salts of Examples 1 to 3 were treated thereto at a concentration of 0.05 mM, 0.1 mM and 0.3 mM, respectively, and then cultured for 20 hours. After culture, the medium was replaced with Phenol Red-free culture medium and then cultured for additional 4 hours. Then, the extracellular lactate in the culture medium, which was released from cells for 4 hours, was evaluated using an assay kit (Biovision, SanFrancisco, CA), and the results are shown in FIG. 3. In this case, cancer cells without any treatment were used as a control.

As shown in FIG. 3, it was confirmed that the lactate concentration released from the cancer cells treated with the calcium salts of Examples 1 to 3 were mostly reduced. Accordingly, it was confirmed that the composition for treating cancer according to the present invention, which comprises an ionic compound combined with a metal ion, can reduce the extracellular lactate concentration released from cancer cells.

### Test Example 1-4: Change in ascorbic acid level

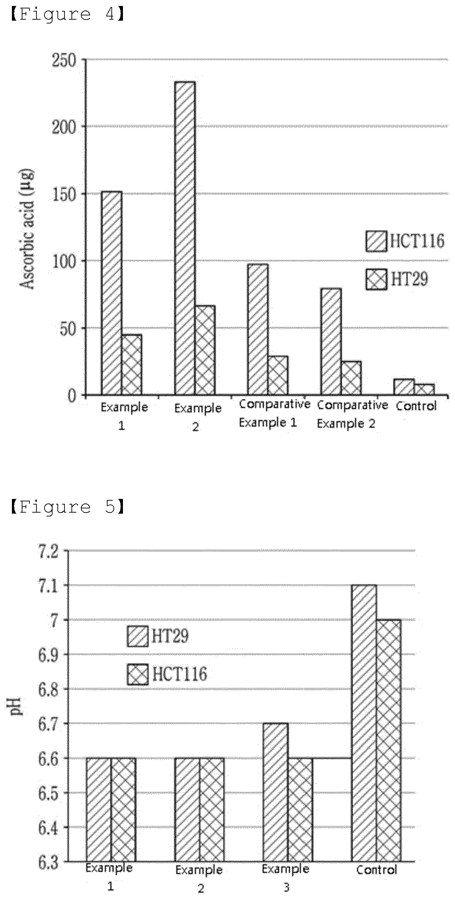

1 mM of the calcium salts of Examples 1 and 2 were treated to human colorectal cancer cell lines (HCT-116 and HT-29) of 5 x 10⁶ cells, which were cultured in cancer cell culture medium (RPMI1640 medium containing 10% FBS and 1% penicillin/streptomycin) at a condition of 37 °C and 5% CO₂, respectively, and then cultured for 24 hours. After culture, the cultured cancer cells were pulverized with a homogenizer and centrifuged, and the concentration of ascorbic acid contained in the lysate was measured using an ascorbic acid assay kit (Biovision, SanFrancisco, CA). The results are shown in FIG. 4. In this case, cancer cells without any treatment were used as a control, cancer cells treated with ascorbic acid (1 mM) were used as Comparative Example 1, and cancer cells treated with calcium ascorbate (1 mM) were used as Comparative Example 2.

As shown in FIG. 4, the ascorbic acid concentration was increased in the cancer cells treated with Examples 1 and 2, while the cancer cells treated with Comparative Examples 1 and 2 had lower level of increased ascorbic acid concentration than the cancer cells treated with Examples 1 and 2. Accordingly, it was confirmed that the composition for treating cancer according to the present invention, which comprises an ionic compound combined with a metal ion, can easily penetrate into cancer cells.

### Test Example 1-5: Change in pH in cancer cell

1 mM of the calcium salts of Examples 1 to 3 were treated to human colorectal cancer cell lines (HCT-116 and HT-29) of 5 x 10⁶ cells, which were cultured in cancer cell culture medium (RPMI1640 medium containing 10% FBS and 1% penicillin/streptomycin) at a condition of 37°C and 5% CO₂, respectively, and then cultured for 24 hours. In the medium of the cultured cells, pH was measured using a pH detection kit (life technologies, CA, and the results are shown in FIG. 5. In this case, cancer cells without any treatment were used as a control.

As shown in FIG. 5, it was confirmed that when the calcium salts of Examples 1 to 3 were treated, intracellular pH was lowered, i.e., acidified. In other words, it was found that the environment in cancer cells was changed to acidic due to the influx of calcium salts. This means that the calcium salts are vulnerable to apoptosis.

### Test Example 2: Effect of calcium salts on metabolism in cancer cells

The calcium salts of Examples 1 to 3 were treated to cancer cells, respectively, to determine their effects on cancer cell metabolism.

### Test Example 2-1: Effect of calcium salts on pyruvate level

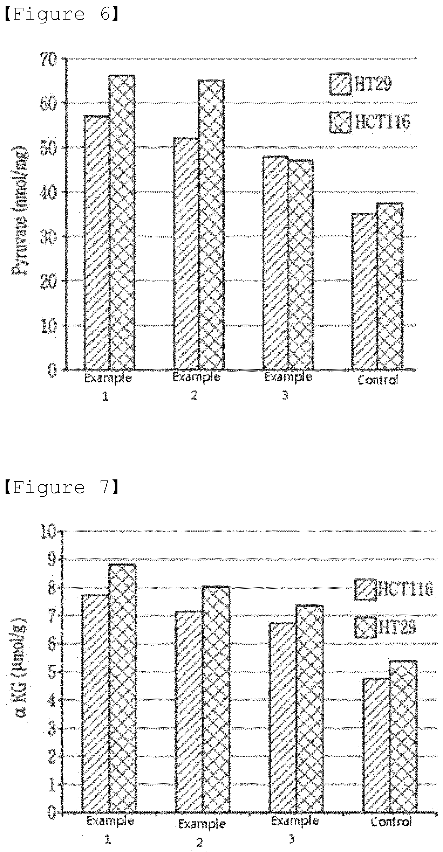

The calcium salts of Example 1 (1 mM), Example 2 (1 mM) and Example 3 (1 mM) were treated to human colorectal cancer cell lines (HCT-116 and HT-29) of 5 x 10⁶ cells, which were cultured in cancer cell culture medium (RPMI1640 medium containing 10% FBS and 1% penicillin/streptomycin) at a condition of 37°C and 5% CO₂, respectively, and then cultured for 24 hours. The cultured cancer cells were pulverized with a homogenizer and centrifuged, and the concentration of pyruvate contained in the lysate was measured using a pyruvate assay kit (Biovision, SanFrancisco, CA). The results are shown in FIG. 6. In this case, cancer cells without any treatment were used as a control.

As shown in FIG. 6, the pyruvate concentration was increased in the cancer cells treated with the calcium salts of Examples 1 to 3. Accordingly, it was confirmed that the composition for treating cancer according to the present invention, which comprises an ionic compound combined with a metal ion, can penetrated into cancer cells and increase the pyruvate concentration.

### Test Example 2-2: Effect of calcium salts on α-ketoglutarate (α-KG) level

The calcium salts of Example 1 (1 mM), Example 2 (1 mM) and Example 3 (1 mM) were treated to human colorectal cancer cell lines (HCT-116 and HT-29) of 5 x 10⁶ cells, which were cultured in cancer cell culture medium (RPMI1640 medium containing 10% FBS and 1% penicillin/streptomycin) at a condition of 37°C and 5% CO₂, respectively, and then cultured for 24 hours. The cultured cancer cells were pulverized with a homogenizer and centrifuged, and the concentration of α-ketoglutarate contained in the lysate was measured using a α-ketoglutarate assay kit (Biovision, SanFrancisco, CA). The results are shown in FIG. 7. In this case, cancer cells without any treatment were used as a control.

As shown in FIG. 7, the α-ketoglutarate concentration was increased in the cancer cells treated with the calcium salts of Examples 1 to 3. Accordingly, it was confirmed that the composition for treating cancer according to the present invention, which comprises an ionic compound combined with a metal ion, can penetrate into cancer cells and induce oxidative phosphorylation process in the mitochondria, thereby increasing the α-ketoglutarate concentration.

### Test Example 2-3: Change in expression level of PARP-1, β-catenin, vascular endothelial growth factor (VEGF) and β-actin protein

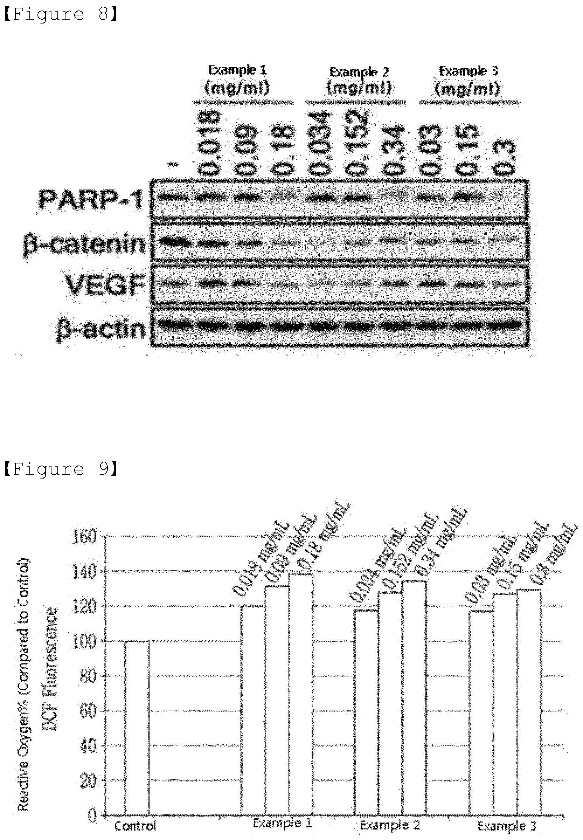

Various concentrations of the calcium salts of Example 1, Example 2 and Example 3 were treated to human colorectal cancer cell line (HCT-116) of 5 x 10⁶ cells, which were cultured in cancer cell culture medium (RPMI1640 medium containing 10% FBS and 1% penicillin/streptomycin) at a condition of 37 °C and 5% CO₂, respectively, and then cultured for 24 hours. The cultured cancer cells were pulverized with a homogenizer and centrifuged, and the expression levels of poly (ADP-ribose) polymerase 1 (PARP-1), β-catenin, VEGF and β-actin proteins contained in the lysate were measured using Western blot. The results are shown in FIG. 8.

As shown in FIG. 8, the expression level of PARP-1 was reduced in the cancer cells treated with the calcium salts of Examples 1 to 3. In general, PARP-1 is used as an apoptosis marker because cleavage occurs by caspase-3, which is activated when cells undergo programmed cell death, i.e., apoptosis. The expression level of full length PAPR-1 was decreased the most at the concentration of 0.18 mg/ml (Example 1), 0.34 mg/ml (Example 2) and 0.3 mg/ml (Example 3). Therefore, it was confirmed that PAPR-1 can induce apoptosis of cancer cells concentration-dependently. Accordingly, it was confirmed that the composition for treating cancer according to the present invention, which comprises an ionic compound combined with a metal ion, can reduce the expression of full length PAPR-1, thereby inducing apoptosis of cancer cells.

Further, it was confirmed that the cancer cells treated with the calcium salts of Examples 1 to 3 decreased the protein level of β-catenin concentration-dependently. β-catenin is a transcription factor that is mutated or over expressed in various carcinomas such as colorectal cancer, lung cancer, breast cancer and ovarian cancer, and is known to regulate the expression of proteins that play an important role in cell growth, cancer metastasis and survival, such as c-myc, cyclin D1, MMP7 and survivin. Accordingly, it was confirmed that the composition for treating cancer according to the present invention, which comprises an ionic compound combined with a metal ion, can suppress cancer cell growth by decreasing the β-catenin protein.

Further, it was confirmed that the cancer cells treated with the calcium salts of Examples 1 to 3 decreased the VEGF expression concentration-dependently. On the other hand, VEGF signaling system plays an important role in cell growth, invasion and metastasis by regulating lower MAPK signaling system and PI3K/Akt signaling system. In particular, it promotes cancer cell metastasis by increasing gene expression of matrix metalloproteinases (MMPs), which is essential for cancer cell metastasis. Accordingly, it was confirmed that the composition for treating cancer according to the present invention, which comprises an ionic compound combined with a metal ion, shows an effect of suppressing cancer cell metastasis by inhibiting the action of factors that induce neovascularization.

### Test Example 2-4: Change in reactive oxygen expression level

In order to measure the concentration change of reactive oxygen in the cancer cells treated with the ionic compound combined with a metal ion according to the present invention, dichlorofluorescin diacetate (DCF-DA; Sigma, USA) was used as a fluorescence probe. DCF-DA is oxidized by ROS in the presence of intracellular hydrogen peroxide-related peroxides and converted to fluorescent DCF, resulting in becoming green fluorescent. Therefore, the measurement of ROS was confirmed through DCF-DA. First, human colorectal cancer cell line (HCT-116) of 5 x 10⁶ cells was cultured in cancer cell culture medium (RPMI1640 medium containing 10% FBS and 1% penicillin/streptomycin) at a condition of 37°C and 5% CO₂, and then cultured for 24 hours. After culture, the cells were washed once with DPBS and culture with DCF-DA 10 µM at 37°C for 30 min. The cells were washed again with DPBS. Various concentrations of calcium salts of Example 1, Example 2 and Example 3 were treated thereto for 6 hours, respectively, and intracellular ROS fluorescence was measured and analyzed. The results are shown in FIG. 9.

As shown in FIG. 9, the cancer cells treated with the calcium salts of Examples 1 to 3 had more reactive oxygen, suggesting the possibility of inducing apoptosis, than the untreated control.

### Test Example 2-5: Change in apoptosis level

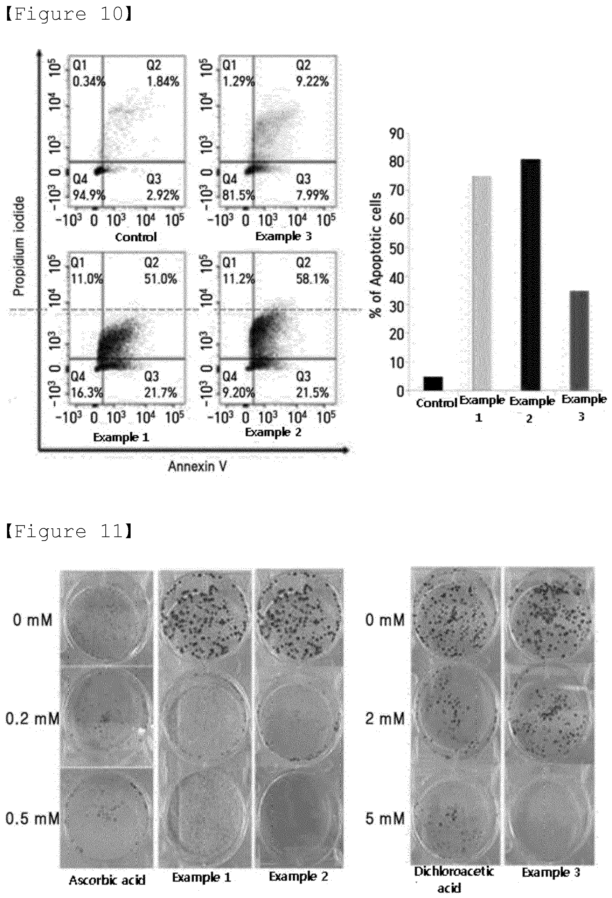

In order to measure the concentration change of reactive oxygen in the cancer cells treated with the ionic compound combined with a metal ion according to the present invention, human colorectal cancer cell line (HCT-116) of 3 x 10⁴ cells was spread on a 6-well plate and cultured for 24 hours. Then, various concentrations of calcium salts of Example 1 (1 mM), Example 2 (1 mM) and Example 3 (1 mM) was treated thereto for 24 hours and washed twice with DPBS. The cells were separated with trypsin-EDTA and stained according to Annexin-V/PI protocol. Apoptosis was measure and analyzed using FACSCanto™ II flow cytometer (Becton-Dickinson, Franklin Lakes, NJ, USA), primary argon laser, and the results are shown in FIG. 10.

In normal living cells, phosphatidyl serine (PS) is located inside the cell membrane. However, at the time of apoptosis, PS is exposed to the outside of the cell membrane, and annexin V binds to PS with high affinity, resulting in fluorescing. Propidium iodide (PI) enters the cell and stains the nucleus. Cells in the early stages of apoptosis are stained only with annexin-V and not with PI, while cells in the later stage of apoptosis or cells undergoing necroses are stained with annexin-V and PI at the same because the integrity of cell membrane is impaired, and no living cells are not stained at all. As shown in FIG. 10, the cancer cells treated with the calcium salts of Examples 1 to 3 were more likely to induce apoptosis than the untreated control.

### Test Example 3: Evaluation of effect on proliferation capacity of cancer cell line

The purpose of this study was to investigate the inhibitory effect on the viability of colorectal cancer, breast cancer and brain cancer cell lines according to the treatment of the calcium salts of Examples 1 to 3.

### Test Example 3-1: Evaluation of effect on proliferation capacity of cancer cell line (MTT assay)

In each well of a 96-well plate, colorectal cancer cell line (DLD-1), breast cancer cell line (MDA-MB-231) and brain cancer cell line (U87MG) were aliquoted at 5 x 10⁶ cells, respectively. The calcium salts of Examples 1 to 3 were added to each well by concentration (20 mg/mℓ, 4 mg/mℓ, 0.8 mg/mℓ, 0.16 mg/mℓ, 0.032 mg/mℓ, 0.0064 mg/mℓ, 0.00128 mg/mℓ, 0.000256 mg/mℓ). For relative comparison, ascorbic acid and dichloroacetic acid were also diluted and added to the wells in the same way. The cells were cultured in an incubator (37°C, 5% CO₂) for 72 hours, and 2 mg/mℓ MTT reagent 50 *µ*ℓ was added thereto and then left in a 37 °C incubator for 4 hours. The supernatant was removed using a centrifuge, DMSO 200 *µ*ℓ was added to each well to dissolve the MTT stained precipitate, and OD₅₄₀ value was measured at 540 nm with an ELISA reader. The 50% inhibitory concentration (IC₅₀) was defined as the concentration of the drug that gave 50% survival rate, and the IC₅₀ value was used as an index of anticancer effect and shown in Table 1 below.

**[Table 1]**

| Section | IC₅₀ of Colorectal cancer cell line (mg/ml) | IC₅₀ of Breast cancer cell line (mg/ml) | IC₅₀ of Brain cancer cell line (mg/ml) |
|---|---|---|---|
| Example 1 | 0.07 | 0.04 | 0.5 |
| Example 2 | 0.07 | 0.05 | 0.6 |
| Example 3 | 0.86 | 0.05 | 0.8 |
| Dichloroacetic acid | 1.21 | 0.43 | 3.71 |
| ascorbic acid | 0.09 | 0.06 | 0.9 |

As show in Table 1, in both calcium salts of Examples 1 and 2, IC₅₀ values for colorectal cancer, breast cancer and brain cancer cell lines were lower than those of ascorbic acid and dichloroacetic acid. Therefore, it was confirmed that they had better cancer cell cytotoxic effect than ascorbic acid and dichloroacetic acid.

In the calcium salts of Example 3, IC₅₀ values for colorectal cancer, breast cancer and brain cancer cell lines were lower than those of dichloroacetic acid. Therefore, it was confirmed that it had better cancer cell cytotoxic effect than dichloroacetic acid.

On the other hand, the calcium salts of Example 3 showed higher IC₅₀ value for colorectal cancer cell line than ascorbic acid, but lower IC₅₀ values for breast cancer and brain cancer cell line than ascorbic acid. Therefore, it was confirmed that it had better breast cancer and brain cancer cell cytotoxic effect than ascorbic acid.

### Test Example 3-2: Evaluation of effect on proliferation capacity of cancer cell line (MTT assay)

Cancer cell proliferation inhibitory capacity of Example 1 and Example 2 was evaluated for 7 cancer cell lines including 2 colorectal cancer cell lines (colorectal cancer cell line (HCT-116, HT-29), lung cancer cell line (A-549), liver cancer cell line (HepG2), pancreatic cancer cell line (PANC-1), stomach cancer cell line (SNU-638) and ovarian cancer cell line (A2780)).

7 cell lines were aliquoted to each well of a 96-well plate at 5 x 10³ cells, and cultured for 24 hours. Then, Example 1 and Example 2 were treated thereto by concentration (5, 2.5, 1.25, 0.625, 0.313, 0.156, 0.078 mM). For relative comparison, ascorbic acid and dichloroacetic acid were also treated in the same way. The drug-treated cells were cultured in an incubator (37 °C, 5% CO₂) for 48 hours, and 5 mg/mℓ MTT reagent 10 *µ*ℓ was added to each well and cultured for additional 4 hours. Then, culture medium was removed and DMSO 100 *µ*ℓ was treated to dissolve the MTT stained precipitate, and absorbance was measured at 540 nm with a microplate reader. The 50% inhibitory concentration (IC₅₀) was defined as the concentration of the drug that gave 50% survival rate, and the IC₅₀ value was used as an index of anticancer effect and shown in Table 2 below.

**[Table 2]**

| Section | IC₅₀ mM of HCT-116 Colorectal cancer cell line (mg/ml) | IC₅₀ mM of HT-29 Colorectal cancer cell line (mg/ml) | IC₅₀ mM of A549 Lung cancer (mg/ml) | IC₅₀ mM of HepG2 Liver cancer (mg/ml) | IC₅₀ mM of PANC-1 Pancreatic cancer (mg/ml) | IC₅₀ mM of SNU-638 Stomach cancer (mg/ml) | IC₅₀ mM of A2780 Ovarian cancer (mg/ml) |
|---|---|---|---|---|---|---|---|
| Example 1 | 0.35 (0.12) | 0.83 (0.29) | 2.3 (0.79) | 2.1 (0.72) | 0.42 (0.14) | 1.40 (0.48) | 0.17 (0.06) |
| Example 2 | 0.33 (0.11) | 0.66 (0.23) | 3.1 (1.06) | 2.3 (0.79) | 0.40 (0.14) | 1.44 (0.49) | 0.14 (0.05) |
| Ascorbic acid | 0.27 (0.09) | 0.93 (0.32) | >5 (>1.7) | 4.2 (1.44) | 0.37 (0.13) | 2.48 (0.85) | 0.15 (0.05) |
| Dichloroacetic acid | >5 (>1.7) | >5 (>1.7) | >5 (>1.7) | >5 (>1.7) | >5 (>1.7) | >5 (>1.7) | >5 (>1.7) |

As shown in Table 2, in addition to colorectal cancer, the calcium salts of Examples 1 and 2 showed anticancer efficacy in lung cancer, liver cancer, pancreatic cancer, stomach cancer and ovarian cancer. Further, it was confirmed that it had better cancer cell cytotoxic effect than dichloroacetic acid because it showed lower IC₅₀ value than dichloroacetic acid.

The calcium salts of Example 1 showed higher IC₅₀ values for colorectal cancer (HCT-116), pancreatic cancer and ovarian cancer cell lines than those of ascorbic acid, but lower IC₅₀ values for colorectal cancer (HT-29), lung cancer, liver cancer and stomach cancer cell lines than those of ascorbic acid. Therefore, it was confirmed that it had better cytotoxic effect for colorectal cancer (HT-29), lung cancer, liver cancer and stomach cancer cells than ascorbic acid.

The calcium salts of Example 2 showed higher IC₅₀ values for colorectal cancer (HCT-116) and pancreatic cancer cell lines than those of ascorbic acid, but lower IC₅₀ values for colorectal cancer (HT-29), lung cancer, liver cancer, stomach cancer and ovarian cancer cell lines than those of ascorbic acid. Therefore, it was confirmed that it had better cytotoxic effect for colorectal cancer (HT-29), lung cancer, liver cancer, stomach cancer and ovarian cancer cells than ascorbic acid.

### Test Example 3-3: Evaluation of effect on colony forming ability of cancer cell line

Human colorectal cancer cell lines (HCT-116) was inoculated to a solid medium containing each of ascorbic acid (0 mM, 0.2 mM, 0.5 mM), calcium salts of Examples 1 and 2 (0 mM, 0.2 mM, 0.5 mM), dichloroacetic acid (0 mM, 2 mM, 5 mM) and calcium salts of Example 3 (0 mM, 2 mM, 5 mM) and cultured for 72 hours. After culture was completed, the cells were fixed and then stained with hematoxylin to observe the cancer cells in which colonies were formed. The result are shown in FIG. 11.

As shown in FIG. 11, all colorectal cancer cell lines not treated with the calcium salts of Examples 1 to 3 formed hundreds of colonies, but as the concentration of each calcium salts of Examples 1 to 3 increased, the number of colonies decreased. Further, the calcium salts of Examples 1 to 3 were more effective in inhibiting the colony forming ability of colorectal cancer than ascorbic acid and dichloroacetic acid treatment. Taken together, it was confirmed that the calcium salts of Examples 1 to 3 showed an inhibitory effect on the colony forming ability of colorectal cancer.

Therefore, summarizing the results of Test Example 3, the composition for treating cancer, which comprises an ionic compound combined to a metal ion according to the present invention can reduce the survival rate of cancer cells such as colorectal cancer, breast cancer and brain cancer.

### Test Example 4: Co-treatment of known anticancer drug and calcium salts of Examples 1 to 3

Known anticancer drug was treated in combination with the calcium salts of Examples 1 to 3 to demonstrate the therapeutic effect on various cancer cell lines.

### Test Example 4-1: Effect of Co-treatment of 5-FU (5-Fluorourasil) and Example

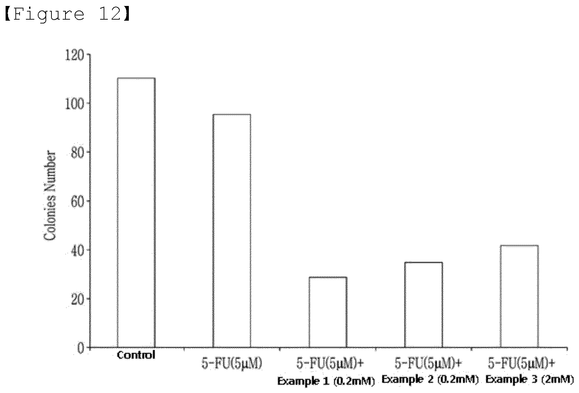

Colorectal cancer cell lines (HCT-116) were aliquoted in each well of a 6-well plate containing RPMI1640 medium at 1 X 10³ cells and after one day, the medium was replaced with fresh medium. Then, the calcium salts of Example 1 (0.2 mM), the calcium salts of Example 2 (0.2 mM), the calcium salts of Example 3 (2 mM) and 5 µM 5-FU were treated alone to each well, and 5 µM 5-FU and the calcium salts of Example 1 (0.2 mM), 5 µM 5-FU and the calcium salts of Example 2 (0.2 mM) and 5 µM 5-FU and the calcium salts of Example 3 (2 mM) were co-treated, respectively. The colony forming abilities were compared and the results are shown in FIG. 12. Human colorectal cancer cell line (HCT-116) not treated with any drug was used as a control.

As shown in FIG. 12, it was observed that the untreated colorectal cancer cell line formed hundreds of colonies, but the case of co-treatment of 5-FU and the calcium salts of Examples 1 to 3 was more effective in inhibiting the colony forming ability of colorectal cancer than 5-FU alone.

### Test Example 4-2: Effect of co-treatment of existing anticancer drug and Example to colorectal cancer cell

HCT-116 cells of 2 X 10³ cells were aliquoted to each well of a 96-well plate and cultured for 24 hours. Then, various concentration of Example or chemical anticancer drugs (Fluorouracil (5-FU), SN-38, Paclitaxel (PTX)) were treated thereto alone. After 48 hours of treatment of each anticancer drug alone, the IC₅₀ value are shown in Table 3.

**[Table 3]**

| **Section** | **HCT-116 cell** | **HT-29 cell** | **DLD-1 cell** |
|---|---|---|---|
| Example 1 | 311 µM | 1,326 µM | 360 µM |
| Example 2 | 430 µM | 1,795 µM | 340 µM |
| Fluorouracil (5-FU) | 6 µM | N.T. | N.T. |
| SN-38 | 0.25 µM | N.T. | N.T. |
| Paclitaxel (PTX) | 7.8 nM | N.T. | N.T. |

| | | | |
|---|---|---|---|
| N.T. (not tested) | | | |

HCT-116 cell of 2 X 10³ cells were aliquoted to each well of a 96-well plate and culture for 24 hours. Then, Example 1 or Example 2 was treated in combination with chemical anticancer drug, Fluorouracil (5-FU), SN-38 or Paclitaxel (PTX) at various concentrations. After 48 hour-exposure, cell growth inhibition rate (%) and co-delivery effect of the co-treated anticancer drug combination were evaluated using the combinational index (CI). As the co-delivery effect, the synergistic effect was CI ≤ 0.85, the additive effect was 0.85 < CI ≤ 1.15, and the antagonistic effect was CI > 1.15. It was confirmed that at most concentrations, the co-treatment of Example 1 or Example 2 and the chemical anticancer drug, Fluorouracil (5-FU), SN-38 and Paclitaxel (PTX) showed synergistic effect.

### Test Example 4-2-1: Effect of co-treatment of Example 1 or Example 2 and Fluorouracil (5-FU)

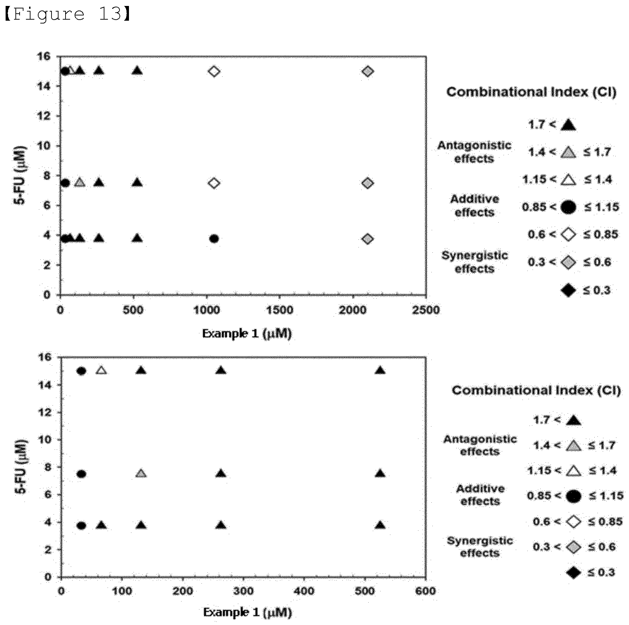

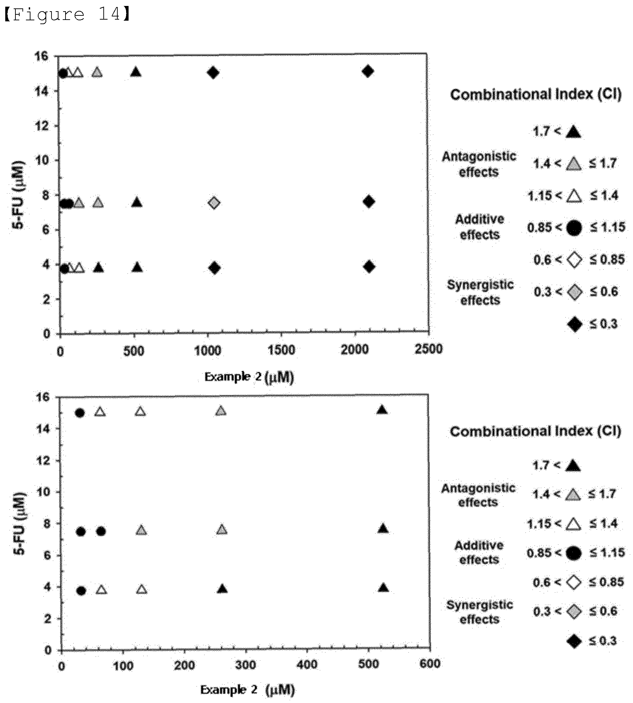

After treating Example 1 and 5-FU, or Example 2 and 5-FU to HCT-116 cells for 48 hours, cell growth inhibition rate (%) and the CI values are shown in Table 4 and Table 5, respectively. The CI value was subdivided into synergistic, additive or antagonistic effect, and are shown in FIG. 13 and FIG. 14, respectively.

When Example 1 or Example 2 was treated with 5-FU at concentration equal to or lower than the IC₅₀ value of Example 1 or Example 2, they showed some antagonistic effect, but when they were co-treated at concentration above 1,000 µM which is higher than the IC₅₀ value of Example 1 or Example 2, they showed synergistic effect mostly at various concentrations.

**[Table 4]**

| **5-FU (µM)** | **Example 1 (µM)** | **Inhibition Effect (%)** | **Combinational Index (CI)** | **Combinational therapeutic effect** |
|---|---|---|---|---|
| 15 | 2100 | 93.577 | 0.4427 | Synergistic |
| 15 | 1050 | 85.3 | 0.6173 | Synergistic |
| 15 | 525 | 58.118 | 1.9302 | Antagonistic |
| 15 | 262.5 | 47.054 | 2.5103 | Antagonistic |
| 15 | 131.25 | 47.275 | 1.9424 | Antagonistic |
| 15 | 65.625 | 50.392 | 1.3341 | Antagonistic |
| 15 | 32.8125 | 52.792 | 1.0142 | Additive |
| 7.5 | 2100 | 94.005 | 0.4066 | Synergistic |
| 7.5 | 1050 | 85.172 | 0.6068 | Synergistic |
| 7.5 | 525 | 51.246 | 2.3129 | Antagonistic |
| 7.5 | 262.5 | 41.521 | 2.4865 | Antagonistic |
| 7.5 | 131.25 | 43.522 | 1.5783 | Antagonistic |
| 7.5 | 32.8125 | 44.392 | 1.0383 | Additive |
| 3.75 | 2100 | 94.164 | 0.3932 | Synergistic |
| 3.75 | 1050 | 78.44 | 0.9802 | Additive |
| 3.75 | 525 | 40.269 | 3.5082 | Antagonistic |
| 3.75 | 262.5 | 35.077 | 2.7914 | Antagonistic |
| 3.75 | 131.25 | 34.349 | 1.9729 | Antagonistic |
| 3.75 | 65.625 | 32.398 | 1.7429 | Antagonistic |
| 3.75 | 32.8125 | 36.111 | 1.0986 | Additive |

**[Table 5]**

| **5-FU (µM)** | **Example 2 (µM)** | **Inhibition Effect (%)** | **Combinational Index (CI)** | **Combinational therapeutic effect** |
|---|---|---|---|---|
| 15 | 2100 | 91.559 | 0.0510 | Synergistic |
| 15 | 1050 | 78.756 | 0.2765 | Synergistic |
| 15 | 525 | 53.337 | 1.9886 | Antagonistic |
| 15 | 262.5 | 51.423 | 1.6698 | Antagonistic |
| 15 | 131.25 | 51.283 | 1.3570 | Antagonistic |
| 15 | 65.625 | 50.93 | 1.2254 | Antagonistic |
| 15 | 32.8125 | 52.97 | 0.9683 | Additive |
| 7.5 | 2100 | 91.863 | 0.0423 | Synergistic |
| 7.5 | 1050 | 72.226 | 0.4879 | Synergistic |
| 7.5 | 525 | 48.921 | 2.2351 | Antagonistic |
| 7.5 | 262.5 | 48.929 | 1.4258 | Antagonistic |
| 7.5 | 131.25 | 44.414 | 1.4760 | Antagonistic |
| 7.5 | 65.625 | 45.648 | 1.0689 | Additive |
| 7.5 | 32.8125 | 44.877 | 0.9954 | Additive |
| 3.75 | 2100 | 92.904 | 0.0294 | Synergistic |
| 3.75 | 1050 | 77.042 | 0.2624 | Synergistic |
| 3.75 | 525 | 47.495 | 2.1676 | Antagonistic |
| 3.75 | 262.5 | 39.686 | 2.4200 | Antagonistic |
| 3.75 | 131.25 | 41.142 | 1.3573 | Antagonistic |
| 3.75 | 65.625 | 38.763 | 1.1837 | Antagonistic |
| 3.75 | 32.8125 | 37.047 | 1.0919 | Additive |

### Test Example 4-2-2: Effect of co-treatment of Example 1 or Example 2 and SN-38

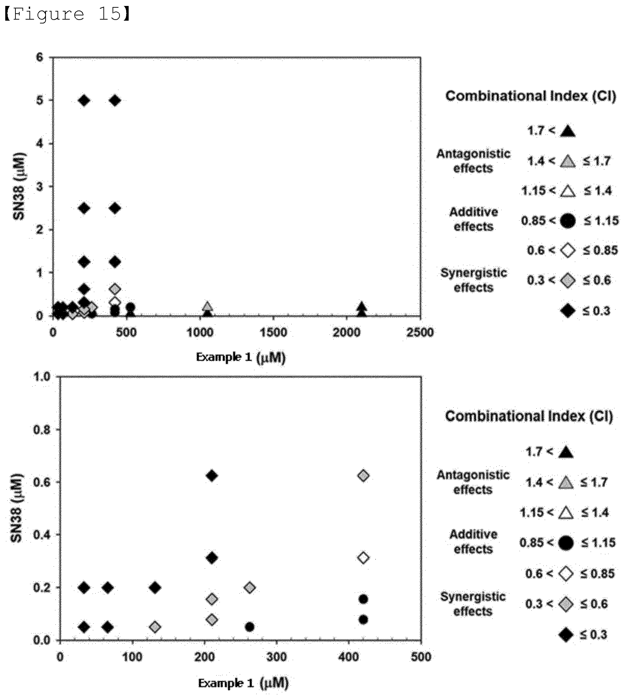

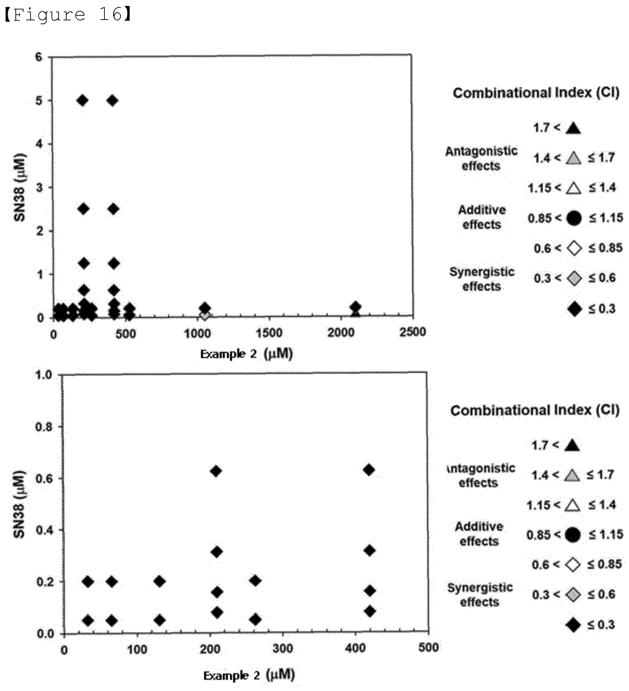

HCT-116 cells were co-treated with Example 1 and SN-38 or Example 2 and SN-38 for 48 hours. The cell growth inhibition rate (%) and the CI value are shown in Table 6 and Table 7, respectively, and the CI value was subdivided into synergistic, additive or antagonistic effect, and are shown in FIG. 15 and FIG. 16, respectively. When the concentration higher than the IC₅₀ value of Example 1 (311 µM) was co-treated with various concentrations of SN-38, the antagonistic effect was observed, but when the concentration near or below the IC₅₀ value of Example 1 was co-treated with various concentrations of SN-38, the synergistic effect was observed. For Example 2, the synergistic effect with SN-38 was observed in most concentration combinations, and especially at combination of the concentration below the IC₅₀ value of Example 2 (430 µM) with the concentration below the IC₅₀ value of SN-38 (0.25 µM), the synergistic effect was also observed.

**[Table 6]**

| **SN-38 (µM)** | **Example 1 (µM)** | **Inhibition Effect (%)** | **Combinational Index (CI)** | **Combinational therapeutic effect** |
|---|---|---|---|---|
| 5 | 420 | 88.9229 | 0.1687 | Synergistic |
| 2.5 | 420 | 92.8722 | 0.0988 | Synergistic |
| 1.25 | 420 | 88.92 | 0.1570 | Synergistic |
| 0.625 | 420 | 79.8278 | 0.3557 | Synergistic |
| 0.3125 | 420 | 70.0759 | 0.6285 | Synergistic |
| 0.1563 | 420 | 62.0811 | 0.9237 | Additive |
| 0.0781 | 420 | 60.9107 | 0.9615 | Additive |
| 5 | 210 | 92.1143 | 0.0564 | Synergistic |
| 2.5 | 210 | 93.5517 | 0.0442 | Synergistic |
| 1.25 | 210 | 89.0431 | 0.0823 | Synergistic |
| 0.625 | 210 | 80.006 | 0.1782 | Synergistic |
| 0.3125 | 210 | 72.083 | 0.2873 | Synergistic |
| 0.1563 | 210 | 65.6346 | 0.3963 | Synergistic |
| 0.0781 | 210 | 64.1575 | 0.4177 | Synergistic |
| 0.2 | 2100 | 80.0428 | 1.7292 | Antagonistic |
| 0.2 | 1050 | 68.4032 | 1.6791 | Antagonistic |
| 0.2 | 525 | 67.5245 | 0.8846 | Additive |
| 0.2 | 262.5 | 66.2025 | 0.4816 | Synergistic |
| 0.2 | 131.25 | 65.0057 | 0.2670 | Synergistic |
| 0.2 | 65.625 | 65.2717 | 0.1420 | Synergistic |
| 0.2 | 32.8125 | 64.9823 | 0.0833 | Synergistic |
| 0.05 | 2100 | 69.6012 | 3.1419 | Antagonistic |
| 0.05 | 1050 | 49.7876 | 3.8746 | Antagonistic |
| 0.05 | 525 | 52.6036 | 1.7291 | Antagonistic |
| 0.05 | 262.5 | 50.4937 | 0.9707 | Additive |
| 0.05 | 131.25 | 52.4243 | 0.4599 | Synergistic |
| 0.05 | 65.625 | 50.3585 | 0.2763 | Synergistic |
| 0.05 | 32.8125 | 46.66 | 0.2076 | Synergistic |

**[Table 7]**

| **SN38 (µM)** | **Example 2 (µM)** | **Inhibition Effect (%)** | **Combinationa 1 Index (CI)** | **Combinational therapeutic effect** |
|---|---|---|---|---|
| 5 | 420 | 91.9418 | 0.0018 | Synergistic |
| 2.5 | 420 | 93.77 | 0.0006 | Synergistic |
| 1.25 | 420 | 90.6416 | 0.0015 | Synergistic |
| 0.625 | 420 | 81.2855 | 0.0092 | Synergistic |
| 0.3125 | 420 | 75.304 | 0.0177 | Synergistic |
| 0.1563 | 420 | 70.6913 | 0.0256 | Synergistic |
| 0.0781 | 420 | 66.2695 | 0.0357 | Synergistic |
| 5 | 210 | 91.8629 | 0.0015 | Synergistic |
| 2.5 | 210 | 93.5454 | 0.0005 | Synergistic |
| 1.25 | 210 | 89.4457 | 0.0014 | Synergistic |
| 0.625 | 210 | 80.0402 | 0.0082 | Synergistic |
| 0.3125 | 210 | 71.5298 | 0.0207 | Synergistic |
| 0.1563 | 210 | 64.2447 | 0.0036 | Synergistic |
| 0.0781 | 210 | 62.6747 | 0.0320 | Synergistic |
| 0.2 | 2100 | 63.246 | 0.2178 | Synergistic |
| 0.2 | 1050 | 66.3675 | 0.0888 | Synergistic |
| 0.2 | 525 | 66.7414 | 0.0510 | Synergistic |
| 0.2 | 262.5 | 64.8357 | 0.0429 | Synergistic |
| 0.2 | 131.25 | 66.2566 | 0.0270 | Synergistic |
| 0.2 | 65.625 | 66.9537 | 0.0204 | Synergistic |
| 0.2 | 32.8125 | 65.6318 | 0.0222 | Synergistic |
| 0.05 | 2100 | 37.5149 | 1.9999 | Antagonistic |
| 0.05 | 1050 | 49.7181 | 0.3514 | Synergistic |
| 0.05 | 525 | 52.0429 | 0.1592 | Synergistic |
| 0.05 | 262.5 | 49.6147 | 0.1242 | Synergistic |
| 0.05 | 131.25 | 51.3344 | 0.0707 | Synergistic |
| 0.05 | 65.625 | 38.8751 | 0.2581 | Synergistic |
| 0.05 | 32.8125 | 42.255 | 0.1 | Synergistic |

### Test Example 4-2-3: Effect of co-treatment of Example 1 or Example 2 and Paclitaxel

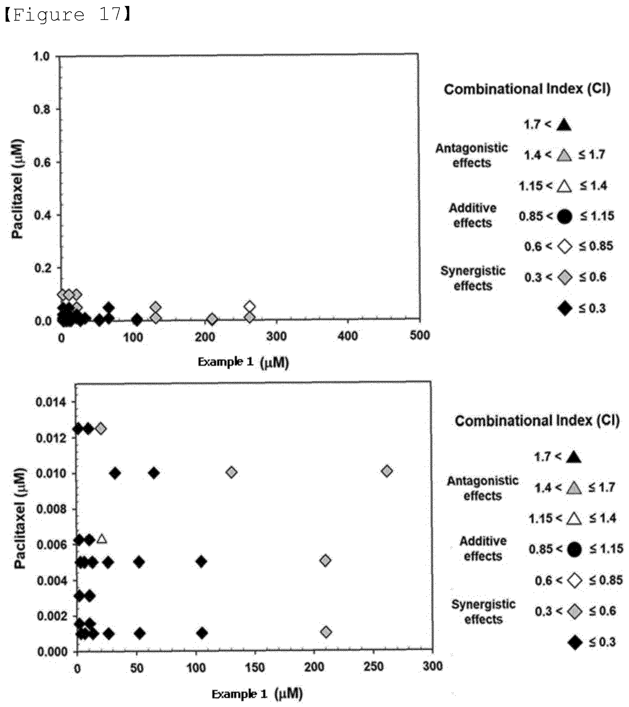

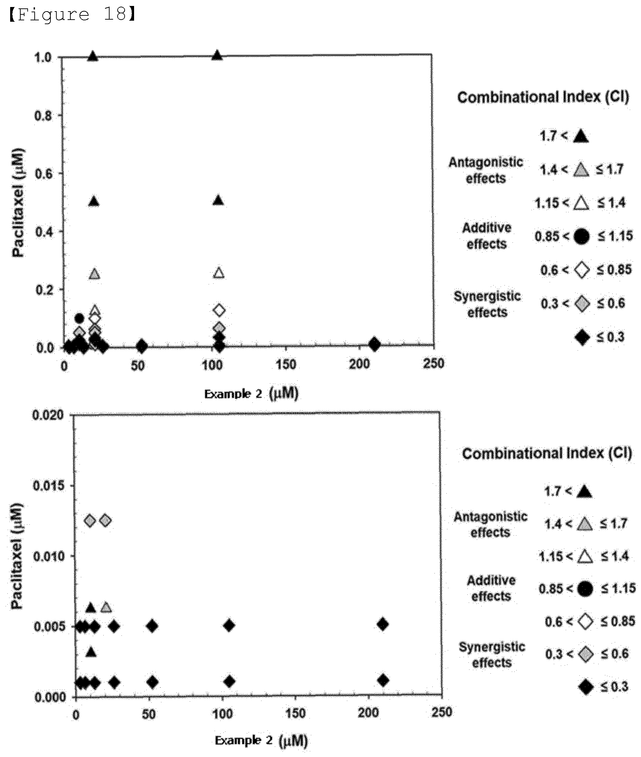

After treating Example 1 and Paclitaxel, or Example 2 and Paclitaxel to HCT-116 cells for 48 hours, cell growth inhibition rate (%) and the CI values are shown in Table 8 and Table 9, respectively. The CI value was subdivided into synergistic, additive or antagonistic effect, and are shown in FIG. 17 and FIG. 18, respectively. When the concentration below the IC₅₀ value of Example 1 (311 µM) or the concentration below the IC₅₀ value of Example 2 (430 µM) and Paclitaxel below 0.1 µM (100 nM) were co-treated, the synergistic effect was observed in most concentration combinations. When the concentration below the IC₅₀ value of Example 1 or Example 2 and the concentration below the IC₅₀ value of Paclitaxel (7.8 nM) were co-treated, the synergistic effect was observed in most concentration combinations.

**[Table 8]**

| **PTX (µM)** | **Example 1 (µM)** | **Inhibition effect (%)** | **Combinational Index (CI)** | **Combinational therapeutic effect** |
|---|---|---|---|---|
| 2000 | 21 | 74.5377 | 4.8733 | Antagonistic |
| 1000 | 21 | 75.7045 | 1.9814 | Antagonistic |
| 500 | 21 | 76.1604 | 0.9214 | Additive |
| 250 | 21 | 75.5324 | 0.5284 | Synergistic |
| 125 | 21 | 74.1361 | 0.3504 | Synergistic |
| 62.5 | 21 | 72.2105 | 0.2551 | Synergistic |
| 31.25 | 21 | 73.5272 | 0.1162 | Synergistic |
| 2000 | 105 | 72.7134 | 6.8186 | Antagonistic |
| 1000 | 105 | 72.7584 | 3.4499 | Antagonistic |
| 500 | 105 | 73.298 | 1.6396 | Antagonistic |
| 250 | 105 | 70.7832 | 1.3048 | Antagonistic |
| 125 | 105 | 70.1536 | 0.7942 | Synergistic |
| 62.5 | 105 | 68.7768 | 0.5640 | Synergistic |
| 31.25 | 105 | 72.8483 | 0.2346 | Synergistic |
| 0.01 | 2100 | 74.538 | 4.8733 | Antagonistic |
| 0.01 | 1050 | 75.705 | 1.9814 | Antagonistic |
| 0.01 | 525 | 76.16 | 0.9214 | Additive |
| 0.01 | 262.5 | 75.532 | 0.5284 | Synergistic |
| 0.01 | 131.25 | 74.136 | 0.3504 | Synergistic |
| 0.01 | 65.625 | 72.211 | 0.2551 | Synergistic |
| 0.01 | 32.8125 | 73.527 | 0.1162 | Synergistic |
| 0.05 | 2100 | 72.758 | 3.4499 | Antagonistic |
| 0.05 | 1050 | 73.298 | 1.6396 | Antagonistic |
| 0.05 | 525 | 70.783 | 1.3048 | Antagonistic |
| 0.05 | 262.5 | 70.154 | 0.7942 | Synergistic |
| 0.05 | 131.25 | 68.777 | 0.5640 | Synergistic |
| 0.05 | 65.625 | 72.848 | 0.2346 | Synergistic |
| 0.1 | 2.1 | 63.569 | 0.4335 | Synergistic |
| 0.05 | 2.1 | 67.66 | 0.1804 | Synergistic |
| 0.025 | 2.1 | 66.42 | 0.1010 | Synergistic |
| 0.0125 | 2.1 | 67.57 | 0.0511 | Synergistic |
| 0.00625 | 2.1 | 65.797 | 0.0338 | Synergistic |
| 0.00313 | 2.1 | 68.827 | 0.0166 | Synergistic |
| 0.00156 | 2.1 | 68.866 | 0.0114 | Synergistic |
| 0.1 | 10.5 | 70.818 | 0.3181 | Synergistic |
| 0.05 | 10.5 | 70.508 | 0.1741 | Synergistic |
| 0.025 | 10.5 | 71.703 | 0.0903 | Synergistic |
| 0.0125 | 10.5 | 70.75 | 0.0603 | Synergistic |
| 0.00625 | 10.5 | 72.076 | 0.0359 | Synergistic |
| 0.00313 | 10.5 | 71.37 | 0.0299 | Synergistic |
| 0.00156 | 10.5 | 71.494 | 0.0250 | Synergistic |
| 0.005 | 210 | 66.813 | 0.8892 | Additive |
| 0.005 | 105 | 69.533 | 0.2995 | Synergistic |
| 0.005 | 52.5 | 66.54 | 0.2454 | Synergistic |
| 0.005 | 26.25 | 60.005 | 0.3220 | Synergistic |
| 0.005 | 13.125 | 46.752 | 0.9711 | Additive |
| 0.1 | 21 | 71.369 | 0.4485 | Synergistic |
| 0.05 | 21 | 66.478 | 0.4952 | Synergistic |
| 0.025 | 21 | 66.499 | 0.2651 | Synergistic |
| 0.0125 | 21 | 57.197 | 0.4975 | Synergistic |
| 0.00625 | 21 | 44.714 | 1.3230 | Antagonistic |
| 0.001 | 210 | 63.569 | 0.4335 | Synergistic |
| 0.001 | 105 | 67.6599 | 0.1804 | Synergistic |
| 0.001 | 52.5 | 66.4198 | 0.1010 | Synergistic |
| 0.001 | 26.25 | 67.5701 | 0.0511 | Synergistic |
| 0.001 | 13.125 | 65.7969 | 0.0338 | Synergistic |
| 0.001 | 6.5625 | 68.8272 | 0.0166 | Synergistic |
| 0.001 | 3.28125 | 68.8664 | 0.0114 | Synergistic |
| 0.005 | 210 | 70.8176 | 0.3181 | Synergistic |
| 0.005 | 105 | 70.5084 | 0.1741 | Synergistic |
| 0.005 | 52.5 | 71.7034 | 0.0903 | Synergistic |
| 0.005 | 26.25 | 70.7495 | 0.0603 | Synergistic |
| 0.005 | 13.125 | 72.0755 | 0.0359 | Synergistic |
| 0.005 | 6.5625 | 71.3732 | 0.0299 | Synergistic |
| 0.005 | 3.28125 | 71.4937 | 0.0250 | Synergistic |
| 0.1 | 10.5 | 66.8129 | 0.8892 | Additive |
| 0.05 | 10.5 | 69.5334 | 0.2995 | Synergistic |
| 0.025 | 10.5 | 66.5395 | 0.2454 | Synergistic |
| 0.0125 | 10.5 | 60.0051 | 0.3220 | Synergistic |
| 0.00625 | 10.5 | 46.7518 | 0.9711 | Additive |

**[Table 9]**

| **PTX (µM)** | **ASCA201 (µM)** | **Inhibition effect (%)** | **Combinational Index (CI)** | **Combinational therapeutic effect** |
|---|---|---|---|---|
| 2000 | 21 | 67.4203 | 15.8618 | Antagonistic |
| 1000 | 21 | 70.5542 | 4.8706 | Antagonistic |
| 500 | 21 | 69.9203 | 2.6664 | Antagonistic |
| 250 | 21 | 68.756 | 1.6081 | Antagonistic |
| 125 | 21 | 66.3676 | 1.1675 | Antagonistic |
| 62.5 | 21 | 66.8142 | 0.5458 | Synergistic |
| 31.25 | 21 | 69.2026 | 0.1881 | Synergistic |
| 2000 | 105 | 74.696 | 4.7156 | Antagonistic |
| 1000 | 105 | 72.0231 | 3.7650 | Antagonistic |
| 500 | 105 | 71.9147 | 1.9194 | Antagonistic |
| 250 | 105 | 70.6184 | 1.1936 | Antagonistic |
| 125 | 105 | 69.9301 | 0.6703 | Synergistic |
| 62.5 | 105 | 69.7345 | 0.3485 | Synergistic |
| 31.25 | 105 | 68.4207 | 0.2177 | Synergistic |
| 0.1 | 10.5 | 65.418 | 1.0785 | Additive |
| 0.05 | 10.5 | 64.7243 | 0.5989 | Synergistic |
| 0.025 | 10.5 | 65.3698 | 0.2722 | Synergistic |
| 0.0125 | 10.5 | 59.8142 | 0.3069 | Synergistic |
| 0.00625 | 10.5 | 40.9398 | 2.0862 | Antagonistic |
| 0.003125 | 10.5 | 32.0304 | 3.9015 | Antagonistic |
| 0.1 | 21 | 68.0909 | 0.7153 | Synergistic |
| 0.05 | 21 | 66.5773 | 0.4656 | Synergistic |
| 0.025 | 21 | 63.4886 | 0.3611 | Synergistic |
| 0.0125 | 21 | 58.1434 | 0.3899 | Synergistic |
| 0.00625 | 21 | 43.5657 | 1.4500 | Antagonistic |
| 0.001 | 210 | 65.3911 | 0.0264 | Synergistic |
| 0.001 | 105 | 65.9839 | 0.0173 | Synergistic |
| 0.001 | 52.5 | 67.8681 | 0.0105 | Synergistic |
| 0.001 | 26.25 | 67.6829 | 0.0092 | Synergistic |
| 0.001 | 13.125 | 67.3283 | 0.0089 | Synergistic |
| 0.001 | 6.5625 | 66.7196 | 0.0093 | Synergistic |
| 0.001 | 3.28125 | 67.6246 | 0.0079 | Synergistic |
| 0.005 | 210 | 67.7855 | 0.0499 | Synergistic |
| 0.005 | 105 | 67.0877 | 0.0484 | Synergistic |
| 0.005 | 52.5 | 67.6759 | 0.0412 | Synergistic |
| 0.005 | 26.25 | 67.9123 | 0.0383 | Synergistic |
| 0.005 | 13.125 | 69.3483 | 0.0299 | Synergistic |
| 0.005 | 6.5625 | 69.5098 | 0.0288 | Synergistic |
| 0.005 | 3.28125 | 68.3333 | 0.0346 | Synergistic |
| 1 | 21 | 70.5542 | 4.8706 | Antagonistic |
| 0.5 | 21 | 69.9203 | 2.6664 | Antagonistic |
| 0.25 | 21 | 68.756 | 1.6081 | Antagonistic |
| 0.125 | 21 | 66.3676 | 1.1675 | Antagonistic |
| 0.0625 | 21 | 66.8142 | 0.5458 | Synergistic |
| 0.0313 | 21 | 69.2026 | 0.1881 | Synergistic |
| 2 | 105 | 74.696 | 4.7156 | Antagonistic |
| 1 | 105 | 72.0231 | 3.7650 | Antagonistic |
| 0.5 | 105 | 71.9147 | 1.9194 | Antagonistic |
| 0.25 | 105 | 70.6184 | 1.1936 | Antagonistic |
| 0.125 | 105 | 69.9301 | 0.6703 | Synergistic |
| 0.0625 | 105 | 69.7345 | 0.3485 | Synergistic |
| 0.0313 | 105 | 68.4207 | 0.2177 | Synergistic |

### Test Example 5: Verification of anticancer effect of calcium salts of Example using animal model

### Test Example 5-1: Construction of carcinoma-formed animal model using animal model (orthotropic model)

In order to construct an animal model with orthotopic xenotransplantation (orthotopic xenograft) and general animal model (orthotopic xenograft), A549/LUC cells and DLD-1 cells were subcultured, and then the cancer cells were injected into the lung and colon of the mice, respectively.

Since the model was directly injected with cancer cells into the mouse organ, the growth of cancer was not confirmed by observation of the mouse appearance. Therefore, in the animal model to which A549/LUC cells were injected, D-Luciferin was injected intraperitoneally every 7 days to determine the growth saturation of cancer by luminescence imaging measurement using IVIS spectrum imaging system (Xenogen) device. On the other hand, in the animal model to which DLD-1 cells were injected, after 7 days, the experimental subjects were sacrificed to determine the growth saturation of cancer. In the animal model to which A549/LUC cells were injected, after about 4 weeks, when the intensity of luminescence was found to be about 10⁷ photons/s/cm²/sr, the calcium salts of Examples 1 to 3 were administered thereto and used for in vivo imaging. In the animal model to which DLD-1 cells were injected, after about 7 weeks, the anticancer effect was observed after administration of the calcium salts of Example 1 at the end stage of colorectal cancer expression.

In other words, general DLD-1 cells were transplanted into the colon to construct a colorectal cancer mouse model (DLD-1 orthotopic model), and orthotopic xenotransplantation was conducted to the lung to construct a lung cancer mouse model (A549/LUC orthotopic model). Then, drugs were administered to each mouse mode as shown in Table 10 below.

**[Table 10]**

| **Animal test model** | **Drug** | **Concentration** | **Drug administration method** | **Population** |
|---|---|---|---|---|
| Mouse model (DLD-1 orthotopic model) | Control | - | Once a day (Qdx4), intravenous injection (IV), for 1 week | 4 |
| Mouse model (DLD-1 orthotopic model) | Example 1 | 100 mg/kg (Dissolved in saline) | Once a day (Qdx4), intravenous injection (IV), for 1 week | 4 |
| Mouse model (A549/LUC orthotopic model) | Control | - | Once a day (Qdx4), intravenous injection (IV), for 5 weeks | 3 |
| Mouse model (A549/LUC orthotopic model) | Example 1 | 100 mg/kg (Dissolved in saline) | Once a day (Qdx4), intravenous injection (IV), for 5 weeks | 3 |
| Mouse model (A549/LUC orthotopic model) | Example 2 | 100 mg/kg (Dissolved in saline) | Once a day (Qdx4), intravenous injection (IV), for 5 weeks | 3 |
| Mouse model (A549/LUC orthotopic model) | Example 3 | 100 mg/kg (Dissolved in saline) | Once a day (Qdx4), intravenous injection (IV), for 5 weeks | 3 |

### Test Example 5-2: Change in anticancer effect and cancer metastasis in animal model injected with calcium salts (1)

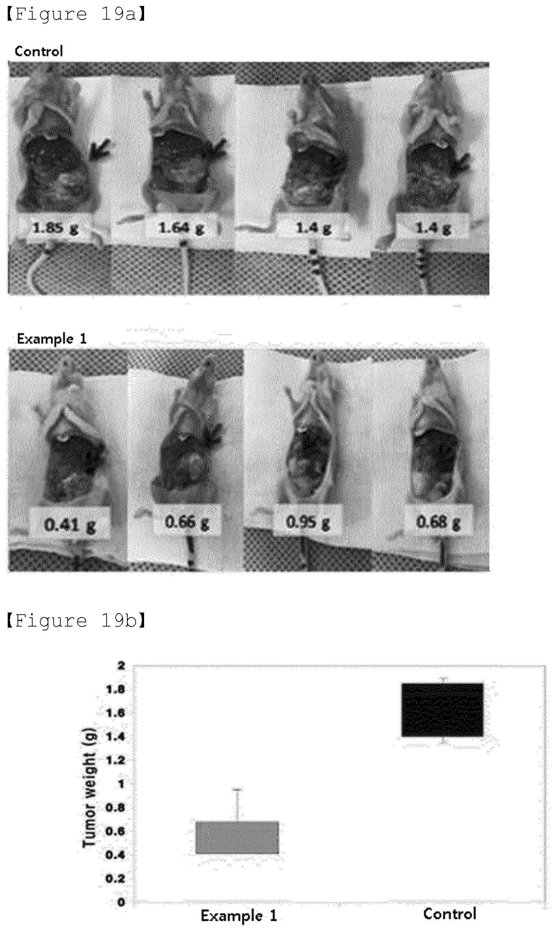

The calcium salts of Example 1 was administered to the same mouse model constructed in Test Example 5-1 (DLD-1 orthotopic model), and dissected after 1 week to observe the growth state of cancer cells. The results are shown in FIG. 19a. Further, weight of cancer tissue was measured to confirm the anticancer efficacy of the inventive substance in vivo and the results are shown in FIG. 19b.

As shown in FIG. 19a and FIG. 19b, all mouse models not treated with the calcium salts of Example 1 (DLD-1 orthotopic model) showed rapid growth of cancer cells, but all mouse models treated with the calcium salts of Example 1 (DLD-1 orthotopic model) showed significantly suppressed growth of cancer cells.

### Test Example 5-3: Change in anticancer effect and cancer metastasis in animal model injected with calcium salts (2)

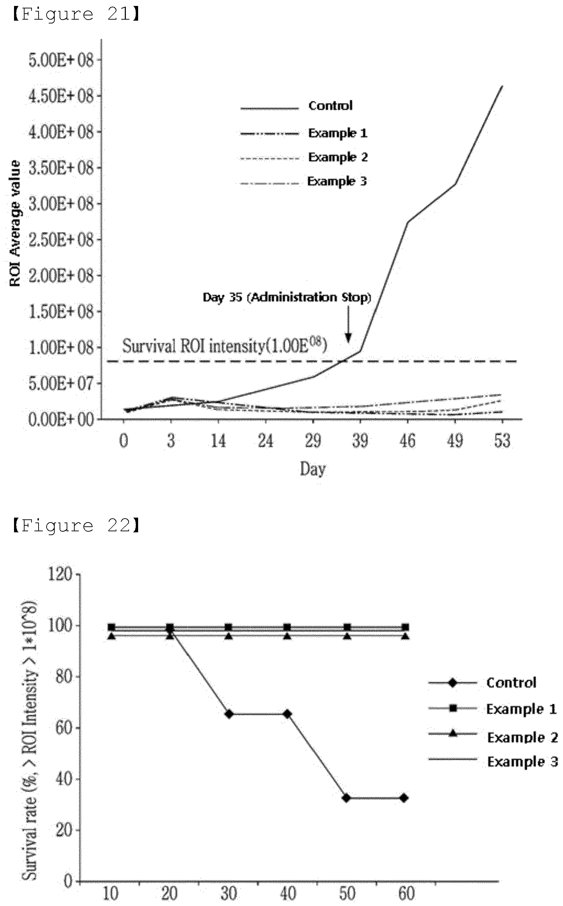

The calcium salts of Examples 1 to 3 were administered to the same mouse model constructed in Test Example 5-1 (A549/LUC orthotopic model), respectively. Then, tissue distribution, metastasis and anticancer efficacy of the invention substance in vivo were confirmed, and images thereof are shown in FIG. Further, in order to quantify each imaging result more accurately, in vivo images were obtained by measuring ROI (Region of Interest), which is a program of IVIS spectrum (Xenogen), and the results are shown in FIG. 21. The survival rate of the mouse model (A549/LUC orthotopic model) was measured and the results are shown in FIG. 22.

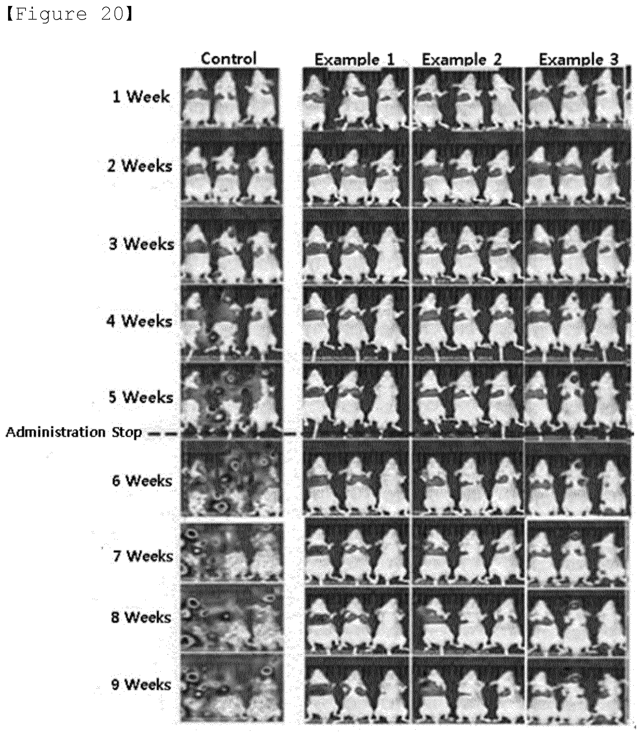

As shown in Figs. 20, 21 and 22, it was confirmed that the calcium salts of Examples 1 to 3 had superior anticancer efficacy, metastasis suppression ability and excellent survival rate compared to the control. In particular, in Example 1, the growth and metastasis of cancer tissues was not shown at all during the drug administration and even after the drug administration was stopped. This suggest that mitochondria in cancer cells have been reformed.

Therefore, through the experimental results described above, it was confirmed that the ionic compound combined with a metal ion according to the present invention can increase uptake of cancer cells. Further, it was confirmed that it can acidify cancer cells by lowering the pH in cancer cells, and the ionic compound in which two compounds were combined with a metal ion was more effective in cancer cell apoptosis than the ionic compound in which one compound (ascorbic acid or dichloroacetic acid) was combined with a metal ion.

Further, it was confirmed that the ionic compound can inhibit aerobic glycolysis of cancer cells by increasing pyruvate and α-ketoglutarate, and can reduce cancer cell proliferation and metastasis by changing the expression level of β-catenin, PARP and VEGF. In addition, through the experiment for the proliferation ability of cancer cell lines, it was confirmed that the anticancer drug can show better anticancer effect when used in combination with the conventional anticancer drugs.

## Claims

1. A pharmaceutical composition for treating cancer, which comprises an ionic compound in which two compounds selected from ascorbic acid, dichloroacetic acid and lactate are combined with one metal ion selected from Ca, Zn, Mg and Fe as an active ingredient.

2. The pharmaceutical composition for treating cancer according to claim 1, wherein the metal ion is Ca²⁺.

3. The pharmaceutical composition for treating cancer according to claim 1, which is used for treatment in combination with radiation or with anticancer drug.

4. The pharmaceutical composition for treating cancer according to claim 3, wherein the radiation is treated in combination with the pharmaceutical composition while irradiating a cancer patient with a dose of 2 to 10 Gy per day.

5. The pharmaceutical composition for treating cancer according to claim 3, wherein the anticancer drug is at least one anticancer drug selected from the group consisting of Imatinib, 5-Florouracil (5-FU), Irinotecan, Sunitinib, Oxaliplatin, Paclitaxel, Lapatinib, Trastuzumab (Herceptin), Gefitinib, Erlotinib, Methotrexate, Carboplatin, Docetaxel, Everolimus, Sorafenib, carbonic anhydrase inhibitor, monocarboxylate transporter inhibitor, Pembrolizumab, Atezolizumab, PD-1 family anticancer drug, Nivolumab, Poly (ADP-ribose) polymerase 1 (PARP-1) inhibitor, Poly (ADP-ribose) polymerase 2 (PARP-2) inhibitor, Olaparib, Rucaparib, Niraparib, Bevacizumab and VEGF inhibitor.

6. The pharmaceutical composition for treating cancer according to claim 1, wherein the cancer is one selected from the group consisting of lung cancer, breast cancer, colorectal cancer, stomach cancer, brain cancer, pancreatic cancer, thyroid cancer, skin cancer, bone marrow cancer, lymphoma, uterine cancer, cervical cancer, kidney cancer and melanoma.

7. The pharmaceutical composition for treating cancer according to claim 1, which further comprises pharmaceutically acceptable carriers, excipients or diluents.

8. The pharmaceutical composition for treating cancer according to claim 1, which is formulated into liquid, powder, aerosol, injection, fluid transfusion (intravenous drip), patch, capsule, pill, tablet, depot or suppository.

9. A pharmaceutical composition for suppressing cancer metastasis, which comprises an ionic compound in which two compounds selected from ascorbic acid, dichloroacetic acid and lactate are combined with one metal ion selected from Ca, Zn, Mg and Fe as an active ingredient.

10. The pharmaceutical composition for suppressing cancer metastasis according to claim 9, wherein the metal ion is Ca²⁺.

11. The pharmaceutical composition for suppressing cancer metastasis according to claim 9, wherein the cancer is one selected from the group consisting of lung cancer, breast cancer, colorectal cancer, stomach cancer, brain cancer, pancreatic cancer, thyroid cancer, skin cancer, bone marrow cancer, lymphoma, uterine cancer, cervical cancer, kidney cancer and melanoma.

12. A pharmaceutical composition for preventing or improving fatigue related to cancer, which comprises an ionic compound in which two compounds selected from ascorbic acid, dichloroacetic acid and lactate are combined with one metal ion selected from Ca, Zn, Mg and Fe as an active ingredient.

13. A food composition for improving cancer, which comprises an ionic compound in which two compounds selected from ascorbic acid, dichloroacetic acid and lactate are combined with one metal ion selected from Ca, Zn, Mg and Fe as an active ingredient.

14. The food composition according to claim 13, which is manufactured as health functional food in the form of cookies, beverages, alcoholic beverages, fermented foods, canned foods, milk-processed foods, meat-processed foods, or noodles.
